# EUROPEAN PATENT APPLICATION

(11) **EP 1 700 596 A1**
(43) Date of publication of application: **13.09.2006**
(21) Application number: 05005151.5
(22) Date of filing: 09.03.2005
(51) Int. Cl.: A61K 31/00, A61K 31/337, A61P 25/00

(54) **Use of microtubule stabilizing compounds for the treatment of lesions of CNS axons**

(71) Applicant: MAX-PLANCK-GESELLSCHAFT ZUR FÖRDERUNG DER WISSENSCHAFTEN E.V., 14195 Berlin (DE)
(72) Inventor: Bradke, Frank, 81375 München (DE); Witte, Harald, 80809 München (DE)
(74) Representative: Vossius & Partner

(57) **Abstract**

The present invention provides a use of one or more microtubule stabilizing compounds for the preparation of a pharmaceutical composition for the treatment of lesions of CNS axons wherein the pharmaceutical composition is administered locally directly into the lesion or immediately adjacent thereto, whereby the one or more compound(s) is/are selected from the group consisting of taxanes, epothilones, laulimalides, sesquiterpene lactones, sarcodictyins, diterpenoids, peloruside A, discodermolide, dicoumarol, ferulenol, NSC12983, taccalonolide A, taccalonolide E, Rhazinilam, nordihydroguaiaretic acid (NDGA), GS-164, borneol esters, Synstab A, Tubercidin and FR182877 (WS9885B). Furthermore, the invention provides a method for the treatment of lesions of CNS axons the method comprising the step of administering to a patient in the need thereof a pharmaceutical composition comprising (i) one or more microtubule stabilizing compounds selected from the group consisting of taxanes, epothilones, laulimalides, sesquiterpene lactones, sarcodictyins, diterpenoids, peloruside A, discodermolide, dicoumarol, ferulenol, NSC12983, taccalonolide A, taccalonolide E, Rhazinilam, nordihydroguaiaretic acid (NDGA), GS-164, borneol esters, Synstab A, Tubercidin and FR182877 (WS9885B) and, optionally, (ii) further comprising suitable formulations of carrier, stabilizers and/or excipients, wherein the pharmaceutical composition is administered locally directly into the lesion or immediately adjacent thereto.

## Description

The present invention relates to a use of one or more microtubule stabilizing compounds for the preparation of a pharmaceutical composition for the treatment of lesions of CNS axons wherein the pharmaceutical composition is administered locally directly into the lesion or immediately adjacent thereto, whereby the one or more compound(s) is/are selected from the group consisting of taxanes, epothilones, laulimalides, sesquiterpene lactones, sarcodictyins, diterpenoids, peloruside A, discodermolide, dicoumarol, ferulenol, NSC12983, taccalonolide A, taccalonolide E, Rhazinilam, nordihydroguaiaretic acid (NDGA), GS-164, borneol esters, SynstabA, Tubercidin and FR182877 (WS9885B). Furthermore, the invention relates to a method for the treatment of lesions of CNS axons the method comprising the step of administering to a patient in the need thereof a pharmaceutical composition comprising (i) one or more microtubule stabilizing compounds selected from the group consisting of taxanes, epothilones, laulimalides, sesquiterpene lactones, sarcodictyins, diterpenoids, discodermolide, dicoumarol, ferulenol, NSC12983, taccalonolide A, taccalonolide E, Rhazinilam, nordihydroguaiaretic acid (NDGA), GS-164, borneol esters, Synstab A, Tubercidin and FR182877 (WS9885B) and, optionally, (ii) further comprising suitable formulations of carrier, stabilizers and/or excipients, wherein the pharmaceutical composition is administered locally directly into the lesion or immediately adjacent thereto.

A variety of documents is cited throughout this specification. The disclosure content of said documents is herewith incorporated by reference.

The loss of sensorimotor functions observed after spinal cord injury follows directly from the limited capacities of the central nervous system (CNS) to regrow its cut axons. The CNS axons fail to regenerate for two reasons. First, the scarring process and the myelin debris create an environment surrounding the damaged area that inhibits axonal growth (4, 22) and, second, the neurons show a poor intrinsic axonal growth in response to injury (1, 16, 23).

Several extra-cellular outgrowth inhibitors have been identified, including myelin proteins such as Nogo, myelin-associated glycoprotein (MAG) and various chondroitin sulfate proteoglycans (CSPGs) (2). Their inhibitory effects occur through neuron specific morphological changes which are characterized by a growth cone collapse and halt of neurite outgrowth (2-4). At the intracellular level, most of these growing inhibitory signals are integrated by changing the activity state of the Rho family of GTPases (2). The members of the Rho GTPases include Cdc42, Rac and Rho. These proteins are involved in remodelling the actin cytoskeleton (5). Axonal growth cone are primarily composed of filamentous actin and microtubules. The microtubules are extending along the entire shaft of the axon and end in the central part of the growth cone. The role of the microtubules in axonal growth will be addressed below and is the major part of our invention. Actin filaments set up the peripheral part of the growth cone as finger-like filopodia and centrally as a thick meshwork called lamellipodia (6). Cdc42 and Rac act on the filopodial and lamellipodial extension respectively, while Rho mediates actin stress fiber formation. In neurons, the activation of Rho leads to growth cone collapse (5). Inversely, inactivation of the whole Rho GTPases with the bacterial toxin B causes a dramatic disruption of the actin network and promotes neurite elongation (7).

The correlation between extra-cellular inhibitors effects and change of Rho GTPases activity state has clearly been recognized. Myelin activates Rho, leading to growth inhibition (8). Conversely, the inactivation of the Rho signalling pathway either by the C3-exoenzyme (9) or by a dominant negative mutation of Rho, promotes neurites growth of neurons cultured on myelin or CSPGs inhibitory substrates (10, 11). Moreover, in vivo, C3-exoenzyme treatment enhances axon regeneration and allows a functional recovery in different models of spinal cord injury (10, 12, 13). Yet, the Rho-GTPases have various other functions ranging from apoptosis to radical formation (14, 15). It is the present understanding in the filed, that lesioned axons in the CNS do not regrow as the corresponding axons in the PNS because the extrinsic inhibitory factors described above activate signalling pathways within the cells that cause the cell to stop axonal growth. The intracellular mechanism underlying axonal growth and axonal halt are not clearly understood.

The initial neuronal polarization of dissociated embryonic hippocampal neurons in cell culture has been analyzed. It was known in the art that shortly after plating four to five neurites per cell form that all of have the potential to become the axon (7, 24, 25). However, only one of the neurites elongates rapidly whereas the other neurites only will grow at a later stage (24). The other neurites are actively restrained from growing: depolymerization of the actin cytoskeleton causes instead of one axon multiple axons to grow (7, 17). Thus it appears that the actin filaments restrain the axons from growing, whereas if the actin filaments are very dynamic and less stable, microtubule can protrude and, thus, allow axonal elongation. Similarly, if the Rho-GTPases are inhibited using the bacterial toxin B hippocampal neurons also form multiple axons per cell (7). Interestingly, as discussed above very similar processes appear to function in lesioned CNS axons (10, 12, 13). Inhibition of Rho using the bacterial toxin C3-exoenzyme causes cut axons to regrow their axon. Indeed using the bacterial toxin C3 exoenzyme is a promising method that probably will move into clinical trials phase I during the years 2004/5. However, a problem with this approach could be that C3 induced depolymerization of the actin filaments may destabilize the tight junctions of the blood brain barrier thereby causing problems.

Even without the understanding of the mechanism underlying axonal growth and axonal halt it has been reported that the treatment of incomplete spinal cord injuries with taxol and methylprednisolone given shortly after a compression injury improve functional outcome in an animal model (41). However, in the treatment required an administration of taxol in a daily dose which was close to the half of the LD10 for daily intraperitoneal doses according to a study by the National Cancer Institute of the USA (42). The problem of the toxicity of taxol in the central nervous system was e.g. confirmed in a recent report in which neuronal death induced by taxol by an induction of apoptotic cell death in an in vivo model was discussed (43).

Thus, the technical problem underlying the present invention was to provide means and methods for the treatment of CNS lesions.

The solution to said technical problem is achieved by providing the embodiments characterized in the claims.

Accordingly, the present invention relates to a use of one or more microtubule stabilizing compounds for the preparation of a pharmaceutical composition for the treatment of lesions of CNS axons wherein the pharmaceutical composition is administered locally directly into the lesion or immediately adjacent thereto, whereby the one or more compound(s) is/are selected from the group consisting of:
taxanes, epothilones, laulimalides, sesquiterpene lactones, sarcodictyins, diterpenoids, peloruside A, discodermolide, dicoumarol, ferulenol, NSC12983, taccalonolide A, taccalonolide E, Rhazinilam, nordihydroguaiaretic acid (NDGA), GS-164, borneol esters, Synstab A, Tubercidin and FR182877 (WS9885B)

The following list provides preferred representatives of microtubule stabilizing compounds envisaged for the use according to the present invention.
- **Taxanes:**
   o Taxol® (Paclitaxel) (Tax-11-en-9-one, 5beta,20-epoxy-1,2alpha,4,7beta, 10beta,13alpha- hexahydroxy-, 4,10-diacetate 2-benzoate, 13-ester with (2R,3S)-N-benzoyl-3-phenylisoserine (8C1)) (Schiff *et al.,* 1979; Schiff and Horwitz, 1980; Wani *et al.,* 1971)
   o IDN5190 (Ortataxel; Hexanoic acid, 3-(((1,1-dimethylethoxy) carbonyl)amino)-2-hydroxy-5-methyl-, (3aS,4R,7R,8aS,9S,10aR,12aS,12bR, 13S,13aS)-7,12a-bis(acetyloxy)-13-(benzoyloxy)-3a,4,7,8,8a,9,10,10a,12,12a, 12b,13-dodecahydro-9-hydroxy-5,8a, 14,14-tetramethyl -2,8-dioxo-6,13a-methano-13aH-oxeto(2",3":5',6')benzo(1',2':4,5)cyclodeca(1,2-d)-1,3-dioxol-4-yl ester, (2R,3S)-) (Nicoletti *et al.,* 2000; Ojima *et al.,* 1996)
   o IDN 5390 (13-(N-BOC-ßisobutylisoserinoyl)-10-dehydro-10-deacetyl-C-secobaccatin) (Taraboletti *et al.,* 2002)
   o BMS-188797 (semi-synthetic derivate of Taxol) (Rose *et al.,* 2001 a)
   o BMS-184476 (7-((Methylthio)methyl)paclitaxel) (Altstadt *et al.,* 2001; Rose *et al.,* 2001 a)
   o BMS-185660 (semi-synthetic derivate of Taxol) (Rose *et al.*, 1997; Rose *et al.,* 2000)
   o RPR109881A (Taxane derivate) (Kurata *et al.,* 2000)
   o TXD258(RPR11625A) (Taxane derivate) (Bissery, 2001; Cisternino *et al.,* 2003)
   o BMS-275183 (orally active taxane) (Rose *et al.,* 2001 b)
   o DJ-927 (orally active taxane) (Shionoya *et al.,* 2003; Syed *et al.,* 2004)
   o Butitaxel analogues (Ali *et al.,* 1997)
   o Macrocyclic Taxane analogues (Tarrant *et al.,* 2004)
   o 7-deoxy-9beta-dihydro-9,10-O-acetal taxanes (Takeda *et al.,* 2003)
   o 10-deoxy-10-C-morpholinoethyl docetaxel analogues (limura *et al.,* 2001)
   o RPR 116258A (Fumoleau *et al.,* 2001; Goetz *et al.,* 2001; Lorthoraly *et al.,* 2000)
   o CT-2103 (Xyotax, PG-TXL) (conjugated poly(L-glutamic acid)-paclitaxel) (Langer, 2004; Li *et al.,* 2000; Li *et al.,* 1998; Multani *et al.,* 1997; Oldham *et al.,* 2000; Todd *et al.,* 2001)
   o polymeric micellar paclitaxel (Leung et *al.,* 2000; Ramaswamy *et al.,* 1997; Zhang *et al.,* 1997a; Zhang *et al.,* 1997b)
   o Genexol-PM, Cremophor-Free, Polymeric Micelle-Formulated Paclitaxel (Kim *et al.,* 2004)
   o Docosahexaenoic acid-conjugated Taxol (Taxoprexin; Paclitaxel 2'-(all-cis-4,7,10,13,16,19-docosahexaenoate)) (Bradley *et al.,* 2001a; Bradley *et al.,* 2001 b; Wolff *et al.,* 2003)
   o PTX-DLPC (Taxol encapsulated into dilauroylphosphatidylcholine liposomal formulations) (Koshkina *et al.,* 2001)
   o PNU-TXL (PNU166945) (a water-soluble polymeric drug conjugate of Taxol) (Meerum Terwogt *et al.,* 2001)
   o MAC-321 (5beta, 20-epoxy-1, 2alpha-, 4-, 7beta-, 10beta-, 13alphahexahydroxytax-11-en-9-one 4 acetate 2 benzoate 7-propionate 13-ester with (2R,3S)-N-tertbutoxycarbonyl-3-(2-furyl)isoserine) (Sampath *et al.,* 2003)
   o Protaxols (water soluble compounds releasing Taxol at basic pH / in plasma) several compounds mentioned in (Nicolaou *et al.,* 1993)
   o photoaffinity analogues of Taxol
      ■ 3'-(p-azidobenzamido)taxol (Rao *et al.,* 1994)
      ■ 2-(m-azidobenzoyl)taxol (Rao *et al.,* 1995)
      ■ 7-(benzoyldihydrocinnamoyl)Taxol (Rao *et al.,* 1999)
      ■ 5-azido-2-nitrobenzoic acid C-7 photoaffinity analogue of Taxol as described in (Carboni *et al.,* 1993)
   o photoactivatable Taxol (2'-(4,5-dimethoxy-2-nitrobenzyl) carbonate of Taxol) (Buck and Zheng, 2002)
   o Docetaxel / Taxotere® (Benzenepropanoic acid, beta-(((1,1-dimethylethoxy) carbonyl)amino)-alpha-hydroxy-, (2aR,4S,4aS,6R,9S, 11 S,12S,12aR,12bS) 12b(acetyloxy)-12-(benzoyloxy)-2a,3,4,4a,5,6,9, 10, 11,12, 12a,12b-dodecahydro-4,6,11-trihydroxy-4a,8,1313-tetramethyl-5-oxo-7,11-methano-1 H-cyclodeca(3,4)benz(1,2-b)oxet-9-yl ester, trihydrate, (alphaR,betaS)-(Ringel and Horwitz, 1991))
- **Epothilones** (Goodin et al., 2004):
   o epothilone A (Bollag *et al.,* 1995; Kowalski *et al.,* 1997b)
   o epothilone B (Patupilone, EPO906) (Bollag *et al.,* 1995; Kowalski *et al.*, 1997b)
   o dEpoB (Chou *et al.,* 2001; Chou *et al.,* 1998a; Chou *et al.,* 1998b)
   o BMS-247550 (aza-EpoB) (Chou *et al.,* 2001; Stachel *et al.,* 2000)
   o F₃-deH-dEpoB (Chou *et al.,* 2003)
   o epothilone D (KOS-862 / NSC-703147) (Dietzmann *et al.,* 2003; Kolman, 2004)
   o dEpoF (Chou *et al.,* 2001)
   o BMS-310705 (21-Aminoepothilone B) (Uyar *et al.,* 2003)
- **Sesquiterpene lactones :**
   o Parthenolide (Germacra-1(10),11(13)-dien-12-oic acid, 4,5-alphaepoxy-6-beta-hydroxy-, gamma-lactone) (Miglietta *et al.,* 2004)
   o Costunolide (Germacra-1(10),4,11(13)-trien-12-oic acid, 6-alphahydroxy-, gamma-lactone, (E,E)-) (Bocca *et al.,* 2004)
- **Sarcodictyins:**
   o Sarcodictyin A (Hamel et al., 1999)
   o Sarcodictyin B (Hamel et al., 1999)
- **Eleutherobins** (Lindel *et al.,* 1997), **caribaeoside and caribaeolin** (Cinel *et al*., 2000)
- **Peloruside A** (secondary metabolite isolated from a New Zealand marine sponge, *Mycale hentscheli)* (Hood *et al.,* 2002)
- **Laulimalide and isolaulimalide** (Mooberry *et al.,* 1999)
- **Discodermolide** (Hung *et al.,* 1996; Kowalski *et al.,* 1997a; Lindel *et al.,* 1997; Martello *et al.,* 2000; ter Haar *et al.,* 1996) (a marine-derived polyhydroxylated alkatetraene lactone, used as a immunosuppressive compound).
   Also envisaged is the use of discodermolide analogues as described in the following publications:
   (a) Hung, D. T.; Nerenberg, J. B.; Schreiber, S. L. Chem. Biol. 1994, 1,67-71.
   (b) Hung, D. T.; Nerenberg, J. B.; Schreiber, S. L. J. Am. Chem. Soc. 1996, 118, 11054-11080.
   (c) Paterson, I.; Florence, G. J. Tetrahedron Lett. 2000, 41, 6935-6939.
   (d) Gunasekera, S. P.; Longley, R. E.; Isbrucker, R. A. J. Nat. Prod. 2001, 64, 171-174.
   (e) Isbrucker, R. A.; Gunasekera, S. P.; Longley, R. E. Cancer Chemother. Pharmacol. 2001, 48, 29-36.
   (f) Martello, L. A.; LaMarche, M. J.; He, L.; Beauchamp, T. J.; Smith, A. B., III; Horwitz, S. B. Chem. Biol. 2001, 8, 843-855. As they were not included in the original paper, full experimental details for the compounds reported therein are provided in the Supporting Information of this work.
   (g) Curran, D. P.; Furukawa, T. Org. Lett. 2002, 4, 2233-2235.
   (h) Gunasekera, S. P.; Longley, R. E.; Isbrucker, R. A. J. Nat. Prod. 2002, 65, 1830-1837.
   (i) Gunasekera, S. P.; Paul, G. K.; Longley, R. E.; Isbrucker, R. A.; Pomponi, S. A. J. Nat. Prod. 2002, 6, 1643-1648.
   (j) Minguez, J. M.; Giuliano, K. A.; Balachandran, R.; Madiraju, C.; Curran, D. P.; Day, B. W. Mol. Cancer Ther. 2002, 1, 1305-1313.
   (k) Shin, Y.; Choy, N.; Balachandran, R.; Madiraju, C.; Day, B. W.; Curran, D. P. Org. Lett. 2002, 4, 4443-4446.
   (I) Choy, N.; Shin, Y.; Nguyen, P. Q.; Curran, D. P.; Balachandran, R.; Madiraju, C.; Day, B. W. J. Med. Chem. 2003, 46, 2846-2864.
   (m) Minguez, J. M.; Kim, S.-Y.; Giuliano, K. A.; Balachandran, R.; Madiraju, C.; Day, B. W.; Curran, D. P. Bioorg. Med. Chem. 2003, 11, 3335-3357.
   (n) Paterson, I.; Delgado, O. Tetrahedron Lett. 2003, 44, 8877-8882.
   (o) Burlingame, M. A.; Shaw, S. J.; Sundermann, K. F.; Zhang, D.; Petryka, J.; Mendoza, E.; Liu, F.; Myles, D. C.; LaMarche, M. J.; Hirose, T.; Freeze, B. S.; Smith, A. B., III. Bioorg. Med. Chem. Lett. 2004, 14, 2335-2338.
   (p) Gunasekera, S. P.; Mickel, S. J.; Daeffler, R.; Niederer, D.; Wright, A. E.; Linley, P.; Pitts, T. J. Nat. Prod. 2004, 67, 749-756.
   (q) Smith, A.B., 3rd, et al., Design, synthesis, and evaluation of analogues of (+)-14-normethyldiscodermolide. Org Lett, 2005. 7(2): p. 315-8.
   (r) Smith, A.B., 3rd, et al., Design, synthesis, and evaluation of carbamate-substituted analogues of (+)-discodermolide. Org Lett, 2005. 7(2): p. 311-4.
- **Dicoumarol** (3,3'-Methylen-bis(4-hydroxy-coumarin)) (Madari *et al.,* 2003)
- **Ferulenol** (prenylated 4-hydroxycoumarin ; 2H-1-Benzopyran-2-one, 4-hydroxy-3-(3,7,11-trimethyl-2,6,10-dodecatrienyl)-) (Bocca *et al.,* 2002))
- **NSC12983** (Wu *et al.,* 2001)
- **Taccalonolides:**
   o taccalonolide A (Tinley et al., 2003)
   o taccalonolide E (Tinley et al., 2003)
- **Rhazinilam** (Indolizino(8,1-ef)(1)benzazonin-6(5H)-one, 8a-ethyl-7,8,8a,9,10,11-hexahydro-, (8aR-(8aR*,14aR*))-) (a plant-derived alkaloid) (David *et al.,* 1994)
- **NDGA and derivates:**
   o Nordihydroguaiaretic acid (NDGA) (4,4'-(2,3-Dimethyl-1,4-butanediyl)bis-1,2-benzenediol) (Nakamura *et al.,* 2003; Smart *et al.,* 1969)
   o Tetra-O-methyl nordihydroguaiaretic acid (Heller *et al.,* 2001)
- **GS-164** (Shintani *et al.,* 1997) (a small synthetic compound)
- **Synstab A** (Haggarty *et al.,* 2000) (a small synthetic compound)
- **borneol esters** (from (Klar *et al.,* 1998), e.g. compound 19a described therein)
- **Tubercidin** (7-beta-CD-ribofuranosyl-7H-pyrrolo(2,3-d)pyrimidin-4-amine) (a nucleoside analog) (Mooberry *et al.,* 1995)
- FR182877 **(WS9885B)** (Sato *et al.,* 2000a; Sato *et al.,* 2000b) (derived from a strain of *Streptomyces sp.* No.9885)

The term "microtubule stabilizing compound" defines in context with the present invention compounds which promote microtubule assembly or inhibit microtubule depolymerization.
The term "lesions of CNS axons" defines in the context of the present invention injuries of CNS axons which comprise spinal cord injuries, neurotraumatic injuries, cut, shot and stab wounds in the nervous system and injuries subsequent of neurodegenerative diseases, multiple sclerosis or stroke.
The local administration directly into the lesion or immediately adjacent thereto has the advantage that severe side effects known to occur concurrently with the systemic administration of microtubule stabilizing compounds do not occur or are observed to a significantly lesser extent.

In accordance with the present invention, the term "pharmaceutical composition" relates to a composition for administration to a patient, preferably a human patient. The pharmaceutical composition is an composition to be administered locally directly into the lesion or immediately adjacent thereto. It is in particular preferred that said pharmaceutical composition is administered to a patient via infusion or injection. Administration of the suitable compositions may also be effected by alternative ways, e.g., by a gelfoam, use of ethylene-vinylacetate copolymers such as Elvax, or by formulation in liposomes, microspheres, nanoparticles or biodegradable polymers.
The pharmaceutical composition of the present invention may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage for continuous infusion might be in the range of 0.01 µg to 2 mg, preferably 0.01 µg to 1 mg, more preferably 0.01 µg to 100 µg, even more preferably 0.01 µg to 50 µg and most preferably 0.01 µg to 10 µg units per point of administration (lesion) per hour.

Preferably the dosage of the microtubule stabilizing compounds envisaged for the use according to the invention is such that the concentrations achieved upon local administration in other body parts, i.e. body parts other than the region to which the local administration is to be effected, do not or not significantly exceed the concentrations in said other body parts achieved upon systemic administration of the exemplary dosages provided in the list below. Thereby it is ensured that toxic side effects known in the art of microtubule stabilizing compounds do not occur or occur in significantly reduced form as compared to the application of doses required for systemic administration in the treatment of CNS lesions (see 41, 43). The skilled person is aware of or capable of determining without further ado the dosages for local administration provided with the information given below. A frequently used indicator is the plasma concentration of a compound. Furthermore, animal experiments are suitable and established in the art to determine said dosage for local administration, wherein said dosage is such that toxic concentrations are not reached in other body parts.
Together with an exemplary dosage for systemic administration the list below also provides corresponding publications.

### • Taxanes

### Taxol

Prescription dose 175 mg/m² i.v. for Paclitaxel ®

### IDN5190

60-90 mg/kg i.v. or p.o. mice
Polizzi, D., Pratesi, G., Tortoreto, M., Supino, R., Riva, A., Bombardelli, E., and Zunino, F. (1999). A novel taxane with improved tolerability and therapeutic activity in a panel of human tumor xenografts. Cancer Res 59, 1036-1040.

### IDN5390

90 mg/kg i.v., s.c. or p.o. mice

Petrangolini G, Cassinelli G, Pratesi G, Tortoreto M, Favini E, Supino R, Lanzi C, Belluco S, Zunino F. Antitumour and antiangiogenic effects of IDN 5390, a novel C-seco taxane, in a paclitaxel-resistant human ovarian tumour xenograft. Br J Cancer. 2004 Apr 5;90(7):1464-8.

### BMS 188797

50 mg/m² i.v. human
Advani R, Fisher GA, Jambalos C, Yeun A, Cho C, Lum BL, et al. Phase I study of BMS 188797, a new taxane analog administered weekly in patients with advanced malignancies. Proceedings of ASCO 2001 ; 20 : 422 (abstr.).

### BMS 184476

60 mg/m² i.v. human
Hidalgo M, Aleysworth C, Hammond LA, Britten CD, Weiss G, Stephenson J Jr, et al. Phase I and pharmacokinetic study of BMS 184476, a taxane with greater potency and solubility than paclitaxel. J Clin Oncol 2001 ; 19 : 2493-503.
Camps C, Felip E, Sanchez JM, Massuti B, Artal A, Paz-Ares L, Carrato A, Alberola V, Blasco A, Baselga J, Astier L, Voi M, Rosell R. Phase II trial of the novel taxane BMS-184476 as second-line in non-small-cell lung cancer. Ann Oncol. 2005 Jan 31

### BMS 185660

48 mg/kg i.v. mice
Rose WC, Clark JL, Lee FY, Casazza AM. Preclinical antitumor activity of water-soluble paclitaxel derivatives. Cancer Chemother Pharmacol. 1997;39(6):486-92.

### RPR 109881A

60-75 mg/m² i.v. human
Kurata T, Shimada Y, Tamura T, Yamamoto N, Hyoda I, Saeki T, et al. Phase I and pharmacokinetic study of a new taxoid, RPR 109881A, given as a 1-hour infusion in patients with advanced solid tumors. J Clin Oncol 2000 ; 18 : 3164-71.
Sessa C, Cuvier C, Caldiera S, Vernillet L, Perard D, Riva A, et al. A clinical and pharmacokinetic (PK) phase I study of RPR 109881A, a new taxoid administerd as a three hour intravenous infusion to patients with advanced solid tumors. Proceedings of ASCO 1998; 17 : 728 (abstr.).

### TXD258(RPR11625A)

40 mg/kg i.v. mice
Gueritte-Voegelein F, Guenard D, Lavelle F, Le Goff MT, Mangatal L, Potier P. Relationships between the structure of taxol analogues and their antimitotic activity. J Med Chem. 1991 Mar;34(3):992-8.

### BMS 275183

6.5-60 mg/kg i.v., p.o. mice
Rose WC, Long BH, Fairchild CR, Lee FY, Kadow JF.Preclinical pharmacology of BMS-275183, an orally active taxane. Clin Cancer Res. 2001 Jul;7(7):2016-21.

### DJ-927

27 mg/m² p.o. in human
Syed, S. K., Beeram, M., Takimoto, C. H., Jakubowitz, J., Kimura, M., Ducharme, M., Gadgeel, S., De Jager, R., Rowinsky, E., and Lorusso, P. (2004). Phase I and Pharmacokinetics (PK) of DJ-927, an oral taxane, in patients (Pts) with advanced cancers. J Clin Oncol (Meeting Abstracts) 22, 2028**.**

9.8 mg/kg/day (8 days) i.v. or p.o. in mice
Shionoya, M., Jimbo, T., Kitagawa, M., Soga, T., and Tohgo, A. (2003). DJ-927, a novel oral taxane, overcomes P-glycoprotein-mediated multidrug resistance in vitro and in vivo. Cancer Sci 94, 459-466.

### Butitaxel analogues

Ali SM, Hoemann MZ, Aube J, Georg Gl, Mitscher LA, Jayasinghe LR. Butitaxel analogues: synthesis and structure-activity relationships. J Med Chem. 1997 Jan 17;40(2):236-41.

### Macrocyclic Taxane analogues

100-200 mg/kg i.v. mice
Tarrant, J. G., Cook, D., Fairchild, C., Kadow, J. F., Long, B. H., Rose, W. C., and Vyas, D. (2004). Synthesis and biological activity of macrocyclic taxane analogues. Bioorg Med Chem Lett 14, 2555-2558.

### 9β-dihydrobaccatin-9,10-acetals derivative taxanes

5.3-40 mg/kg p.o., i.v. mice
Takeda Y, Yoshino T, Uoto K, Chiba J, Ishiyama T, Iwahana M, Jimbo T, Tanaka N, Terasawa H, Soga T. New highly active taxoids from 9betadihydrobaccatin-9,10-acetals. Part 3. Bioorg Med Chem Lett. 2003 Jan 20; 13(2): 185-90.

### 10-deoxy-10-C-morpholinoethyl docetaxel analogues

112.5-600 mg/kg i.v., p.o. mice
limura S, Uoto K, Ohsuki S, Chiba J, Yoshino T, lwahana M, Jimbo T, Terasawa H, Soga T. Orally active docetaxel analogue: synthesis of 10-deoxy-10-C-morpholinoethyl docetaxel analogues. Bioorg Med Chem Lett. 2001 Feb 12;11 (3):407-10.

### RPR 116258A

8-25 mg/m² i.v. human

Lorthoraly A, Pierga JY, Delva R, Girre V, Gamelin E, Terpereau A, et al. Phase I and pharmacokinetic (PK) study of RPR 116258A given as a 1-hour infusion in patients (pts) with advanced solid tumors. Proceedings of the 11th NCI-EORTC-AACR Symposium 2000 (résumé 569) : 156-7.

Goetz AD, Denis LJ, Rowinsky EK, Ochoa L, Mompus K, Deblonde B, et al. Phase I and pharmacokinetic study of RPR 116258A, a novel taxane derivative, administered intravenously over 1 hour every 3 weeks. Proceedings of ASCO 2001 ; 20 : 419 (abstr.), 106a.

Fumoleau P, Trigo J, Campone M, Baselga J, Sistac F, Gimenez L, et al. Phase I and pharmacokinetic (PK) study of RPR 116258A, given as a weekly 1-hour infusion at day 1, day 8, day 15, day 22 every 5 weeks in patients (pts) with advanced solid tumors. Proceedings of the 2001 AACR-NCI-EORTC International Conference. Résumé 282 : 58.

### PG-TXL =CT-2103 = XYOTAX

175 mg/m² i.v. human
Langer CJ. CT-2103: a novel macromolecular taxane with potential advantages compared with conventional taxanes. Clin Lung Cancer. 2004 Dec;6 Suppl 2:S85-8.

### Polymeric micellar paclitaxel

25 mg/kg i.v. mice
Zhang X, Burt HM, Mangold G, Dexter D, Von Hoff D, Mayer L, Hunter WL. Anti-tumor efficacy and biodistribution of intravenous polymeric micellar paclitaxel. Anticancer Drugs. 1997 Aug;B(7):696-701.

### Genexol-PM, Cremophor-Free, Polymeric Micelle-Formulated Paclitaxel

135-390 mg/m² i.v. human
Kim TY, Kim DW, Chung JY, Shin SG, Kim SC, Heo DS, Kim NK, Bang YJ. Phase I and pharmacokinetic study of Genexol-PM, a cremophor-free, polymeric micelle-formulated paclitaxel, in patients with advanced malignancies. Clin Cancer Res. 2004 Jun 1;10(11):3708-16.

### Docosahexaenoic acid-coniugated Taxol (Taxoprexin)

60-120 mg/kg i.v. mice
Bradley MO, Webb NL, Anthony FH, Devanesan P, Witman PA, Hemamalini S, Chander MC, Baker SD, He L, Horwitz SB, Swindell CS. Tumor targeting by covalent conjugation of a natural fatty acid to paclitaxel. Clin Cancer Res. 2001 Oct;7(10):3229-38.

### PTX-DLPC (aerosol)

5mg/kg inhalation mice
Koshkina NV, Waldrep JC, Roberts LE, Golunski E, Melton S, Knight V. Paclitaxel liposome aerosol treatment induces inhibition of pulmonary metastases in murine renal carcinoma model. Clin Cancer Res. 2001 Oct;7(10):3258-62.

### PNU-TXL

80-196 mg/m² i.v. human
Meerum Terwogt JM, ten Bokkel Huinink WW, Schellens JH, Schot M, Mandjes IA, Zurlo MG, Rocchetti M, Rosing H, Koopman FJ, Beijnen JH.Phase I clinical and pharmacokinetic study of PNU166945, a novel water-soluble polymer-conjugated prodrug of paclitaxel. Anticancer Drugs. 2001 Apr;12(4):315-23.

### MAC-321

10-70mg/kg i.v. in mice
Lethal dose 140mg/kg
Sampath, D., Discafani, C. M., Loganzo, F., Beyer, C., Liu, H., Tan, X., Musto, S., Annable, T., Gallagher, P., Rios, C., and Greenberger, L. M. (2003). MAC-321, a novel taxane with greater efficacy than paclitaxel and docetaxel in vitro and in vivo. Mol Cancer Ther 2, 873-884.

### Docetaxel/Taxotere

Prescription dose 100 mg/m² i.v. for Taxotere®
75 mg/m² i.v. human according to Kulke MH, Kim H, Stuart K, Clark JW, Ryan DP, Vincitore M, Mayer RJ, Fuchs CS. A phase II study of docetaxel in patients with metastatic carcinoid tumors. Cancer Invest. 2004;22(3):353-9.

### • Epothilon

### Epothilone B (Patupilone, EPO906)

2-4 mg/kg mice in combination with the protein kinase inhibitor imatinib (STI 571, Glivec)
O'Reilly, T., Wartmann, M., Maira, S. M., Hattenberger, M., Vaxelaire, J., Muller, M., Ferretti, S., Buchdunger, E., Altmann, K. H., and McSheehy, P. M. (2005). Patupilone (epothilone B, EPO906) and imatinib (STI571, Glivec) in combination display enhanced antitumour activity in vivo against experimental rat C6 glioma. Cancer Chemother Pharmacol 55, 307-317.

### Desoxyepothilone B

15-60 mg/kg i.v. mice
Chou TC, Zhang XG, Harris CR, Kuduk SD, Balog A, Savin KA, Bertino JR, Danishefsky SJ. Desoxyepothilone B is curative against human tumor xenografts that are refractory to paclitaxel. Proc Natl Acad Sci U S A. 1998 Dec 22;95(26):15798-802.

### BMS-310705 (Aza-EpoB)

40 mg/m² i.v. human
Eng C, Kindler HL, Nattam S, Ansari RH, Kasza K, Wade-Oliver K, Vokes EE.A phase II trial of the epothilone B analog, BMS-247550, in patients with previously treated advanced colorectal cancer. Ann Oncol. 2004 Jun;15(6):928-32.

### F3-deH-dEpoB

20-30 mg/kg i.v. mice
Chou TC, Dong H, Rivkin A, Yoshimura F, Gabarda AE, Cho YS, Tong WP, Danishefsky SJ. Design and total synthesis of a superior family of epothilone analogues, which eliminate xenograft tumors to a nonrelapsable state. Angew Chem Int Ed Engl. 2003 Oct 13;42(39):4762-7.

### Epothilone D

Kolman, A. (2004). Epothilone D (Kosan/Roche). Curr Opin Investig Drugs 5, 657-667.

### dEpoF

15-30 mg/kg
Chou TC, O'Connor OA, Tong WP, Guan Y, Zhang ZG, Stachel SJ, Lee C, Danishefsky SJ. The synthesis, discovery, and development of a highly promising class of microtubule stabilization agents: curative effects of desoxyepothilones B and F against human tumor xenografts in nude mice. Proc Natl Acad Sci USA. 2001 Jul 3;98(14):8113-8.

### • Sesquiterpene lactones

### Parthenolide

1-5 mg/day (tablets) p.o. human
Curry EA 3rd, Murry DJ, Yoder C, Fife K, Armstrong V, Nakshatri H, O'Connell M, Sweeney CJ. Phase I dose escalation trial of feverfew with standardized doses of parthenolide in patients with cancer. Invest New Drugs. 2004 Aug;22(3):299-305.

### Costunolide

50mg/kg p.o. rats
Matsuda H, Shimoda H, Ninomiya K, Yoshikawa M. Inhibitory mechanism of costunolide, a sesquiterpene lactone isolated from Laurus nobilis, on blood-ethanol elevation in rats: involvement of inhibition of gastric emptying and increase in gastric juice secretion. Alcohol Alcohol. 2002 Mar-Apr;37(2):121-7.

### • Dicoumarol

34 mg/kg i.p. mice
Begleiter A, Leith MK, Thliveris JA, Digby T. Dietary induction of NQO1 increases the antitumour activity of mitomycin C in human colon tumours in vivo. Br J Cancer. 2004 Oct 18;91 (8):1624-31.

### • Ferulenol

2-319 mg/kg i.p. or p.o. mice toxicity test...
Fraigui O, Lamnaouer D, Faouzi MY. Acute toxicity of ferulenol, a 4-hydroxycoumarin isolated from Ferula communis L. Vet Hum Toxicol. 2002 Feb;44(1):5-7.

### • NDGA derivative Tetra-O-methyl nordihydroguaiaretic acid

20 mg intra-tumoral mice
Heller JD, Kuo J, Wu TC, Kast WM, Huang RC. Tetra-O-methyl nordihydroguaiaretic acid induces G2 arrest in mammalian cells and exhibits tumoricidal activity in vivo. Cancer Res. 2001 Jul 15;61 (14):5499-504.

### • Tubercidin

10-50 mg/kg p.o., i.v. mice
Olsen DB, Eldrup AB, Bartholomew L, Bhat B, Bosserman MR, Ceccacci A, Colwell LF, Fay JF, Flores OA, Getty KL, Grobler JA, LaFemina RL, Markel EJ, Migliaccio G, Prhavc M, Stahlhut MW, Tomassini JE, MacCoss M, Hazuda DJ, Carroll SS. A 7-deaza-adenosine analog is a potent and selective inhibitor of hepatitis C virus replication with excellent pharmacokinetic properties. Antimicrob Agents Chemother. 2004 Oct;48(10):3944-53.
Jaffe JJ, Doremus HM, Meymarian E. Activity of tubercidin against immature Fasciola hepatica in mice. J Parasitol. 1976 Dec;62(6):910-3.

### • FR182877

Sato B, Muramatsu H, Miyauchi M, Hori Y, Takase S, Hino M, Hashimoto S, Terano H. A new antimitotic substance, FR182877. I. Taxonomy, fermentation, isolation, physico-chemical properties and biological activities. J Antibiot (Tokyo). 2000 Feb;53(2):123-30.
Sato, B., Nakajima, H., Hori, Y., Hino, M., Hashimoto, S., and Terano, H. (2000b). A new antimitotic substance, FR182877. II. The mechanism of action. J Antibiot (Tokyo) 53, 204-206.

Particularly preferred dosages are recited herein below. Progress can be monitored by periodic assessment.
Preparations for parenteral administration include sterile aqueous or non-aqueous solutions, suspensions, and emulsions. Examples of non-aqueous solvents are propylene glycol, polyethylene glycol, vegetable oils such as olive oil, injectable organic esters such as ethyl oleate, and further organic solvents including DMSO. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, fixed oils or organic solvents including DMSO. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition might comprise proteinaceous carriers, like, e.g., serum albumin or immunoglobulin, preferably of human origin. It is envisaged that the pharmaceutical composition of the might comprise, in addition to the microtubule stabilizing compound, further biologically active agents, depending on the intended use of the pharmaceutical composition. Such agents might be drugs acting as cytostatica, drugs preventing hyperurikemia (decreased renal excretion of uric acid), drugs inhibiting immunereactions (e.g. corticosteroids), and/or drugs acting on the circulatory system.
The administration of the pharmaceutical compound described herein is defined as locally directly into the lesion or immediately adjacent thereto. This definition is understood in the context of the present invention as an opposite to a more widespread or systemic administration. Such local administration may be effected e.g. by an injection or continuous infusion of the pharmaceutical composition directly into the lesion or immediately adjacent thereto. Alternatively, the composition may be administered e.g. by means of gelfoam or Elvax which are known in the art, inter alia from (44).

It has been surprisingly found that the microtubules of the axon in polarized neurons during initial axon formation show a higher ratio of acetylated/ tyrosinated tubulin and that the microtubules in the future axons are more resistant to nocodazol depolymerization. Furthermore, stabilization of microtubules using taxol, a drug used in cancer treatment, causes multiple axons to grow.

Beside of this effect of microtubule stabilizing which is the unique feature of the above recited compounds, taxol treated cultures show a drastic reduction in astrocyte numbers in these cultures. This induction of cell death was understood to be the effect of taxol to kill dividing cells.
Without being bound by theory, the combination of the microtubule stabilizing effect of taxol with the effect of drastic reduction in astrocyte numbers may result in an additional improvement of the treatment of CNS lesions. This additional benefit may be explained by an induction of intrinsic growth capacity (stabilizing the microtubules of the axons and thereby inducing axonal regrowth) and, second, a reduction of the inhibitory environment of the glial scar by reducing the astrocytes at the lesion site.

The present invention furthermore relates to a method for the treatment of lesions of CNS axons the method comprising the step of administering to a patient in the need thereof a pharmaceutical composition comprising
(i) one or more microtubule stabilizing compounds selected from the group consisting of taxanes, epothilones, laulimalides, sesquiterpene lactones, sarcodictyins, diterpenoids, peloruside A, discodermolide, dicoumarol, ferulenol, NSC12983, taccalonolide A, taccalonolide E, Rhazinilam, nordihydroguaiaretic acid (NDGA), GS-164, borneol esters, SynstabA, Tubercidin and FR182877 (WS9885B) and, optionally,
(ii) further comprising suitable formulations of carrier, stabilizers and/or excipients,
wherein the pharmaceutical composition is administered locally directly into the lesion or immediately adjacent thereto.

According to the use and the method of the invention it is preferred that
(a) the taxane(s) is/are selected from the group consisting of IDN5190, IDN 5390, BMS-188797, BMS-184476, BMS-185660, RPR109881A, TXD258(RPR11625A), BMS-275183, PG-TXL, CT-2103, polymeric micellar paclitaxel, Taxoprexin, PTX-DLPC, PNU-TXL (PNU166945), MAC-321, Taxol, Protaxols, photoaffinity analogues of Taxol, photoactivatable Taxol and Docetaxel/Taxotere;
(b) the epothilone(s) is/are selected from the group consisting of epothilone A, epothilone B, dEpoB, BMS-247550 (aza-EpoB), epothilone D, dEpoF and BMS-310705;
(c) the sesquiterpene lactone(s) is/are Parthenolide or Costunolide;
(d) the sarcodictyin(s) is/are selected from sarcodictyin A and sarcodictyin B;
(e) the diterpenoid(s) is/are selected from the group consisting of Eleutherobins, caribaeoside and caribaeolin; or
(f) the discodermolide is/are a marine-derived polyhydroxylated alkatetraene lactone, used as a immunosuppressive compound.

Protaxols are known as water soluble compounds releasing taxol at basic pH/in plasma (Nicolaou et al., 1993). Photoaffinity analogues of taxol are described in Rao et al., 1999; Rao et al., 1994. Photoactivatable taxol is known from Buck and Zheng, 2002. Docetaxel/Taxotere is known from Ringel and Horwitz, 1991. Parthenolide is known from Miglietta et al., 2004. Eleutherobin is known from Long et al., 1998. epothilones A and B are known from Ojima et al., 1999 and epothilone D from Dietzmann et al., 2003. Dicoumarol is known from Madari et al., 2003. Ferulenol (a prenylated 4-hydroxycoumarin) is known from Bocca et al., 2002. Nordihydroguaiaretic acid (NDGA) is known from Nakamura et al., 2003. BMS-310705 is known from Uyar et al., 2003. NSC12983 (synthetic steroid derivative) is known from Wu et al., 2001. IDN 5390 is known from Taraboletti et al., 2002. Sarcodictyin A and B is known from Hamel et al., 1999. GS-164 (a small synthetic compound) is known from Shintani et al., 1997. Borneol esters suitable as inhibitors of microtubule depolymerization are known from Klar et al., 1998. Particularly envisaged for the use according to the present invention is compound 19a described in Klar et al., 1998. Compounds isolated from the octocoral Erythropodium caribaeorum such as eleutherobin, the new antimitotic diterpenoids desmethyleleutherobin, desacetyleleutherobin, isoeleutherobin A, Z-eleutherobin, caribaeoside, and caribaeolin are known from Cinel et al., 2000. Tubercidin (7-deazaadenosine) is known from Mooberry et al., 1995. Taccalonolides (plant-derived steroids with microtubule-stabilizing activity) are known from Tinley et al., 2003. Rhazinilam, a plant-derived alkaloid, is known from David et al., 1994. Costunolide, a sesquiterpene lactone found in medicinal herbs, is known from Bocca et al., 2004.

As described herein above, the pharmaceutical composition may be locally administered by different means and methods. According to a preferred embodiment of the invention the pharmaceutical composition is administered using an osmotic pump. Osmotic pumps are infusion pumps for continuos dosing. Osmotic pumps and their uses are generally known to the person skilled in the art. Osmotic pumps may be obtained from a variety of manufacturers including Alzet (www.alzet.com). Alternatively, administration is to be effected by local syringe injection. The envisaged routes of administration imply appropriate formulations. The skilled person is aware of formulations suitable for the above mentioned routes of administration.
In a further preferred embodiment, said pharmaceutical composition comprises gelfoam, Elvax, liposomes, microspheres, nanoparticles and/or biodegradable polymers.

In a more preferred embodiment of the invention the one or more taxanes comprised in the pharmaceutical composition is/are in a final concentration in a range of 0,1 pM and 1 mM. More preferably, said final concentration is in a range of 0.1 pM to 100µM, 0.1µM to 1µM, 0.1µM to 100nM, 0.1µM to 10µM, 0.1µM to 1µM, or 10µM to 1 nM, and most preferred between 100µM and 1nM.

It is also envisaged by the present invention that the pharmaceutical composition further comprises a compound selected from the group of c3-exoenzyme, chondroitinase, an iron chelator, cAMP and its derivatives such as dibutyryl cAMP (as described in Neumann et al., 2002), and substances that increase the intracellular cAMP level, either by stimulating the adenyl cyclase or by inhibiting phosphodiesterase, including rolipram. Said chelator is to be administered at the lesion site. cAMP and its derivatives such as dibutyryl cAMP and phosphdoesterase inhibitors such as rolipram may be administered locally at the lesion site or at the location of the brain nuclei or ganglia (location of the cell body) of the neural class to be treated, or systemically by infusion. Optionally, the pharmaceutical composition may further comprise suitable formulations of carrier, stabilizers and/or excipients.
The beneficial effects of c3-exoenzyme and chondroitinase on axonal growth are known in the art (see references 10 and 12 as regards c3-exoenzyme and
Bradbury et al., 2002 and Moon et al., 2001 as regards chondroitinase) and the compounds are the subject of clinical studies. Iron chelator therapy and cAMP therapy are also known to the person skilled the art e.g. from Hermanns et al., 2001 (iron chelator therapy) and from Nikulina et al., 2004; Pearse et al., 2004; and Neumann et al., 2002 (therapy with cAMP derivatives and/or rolipram).

### The Figures show:

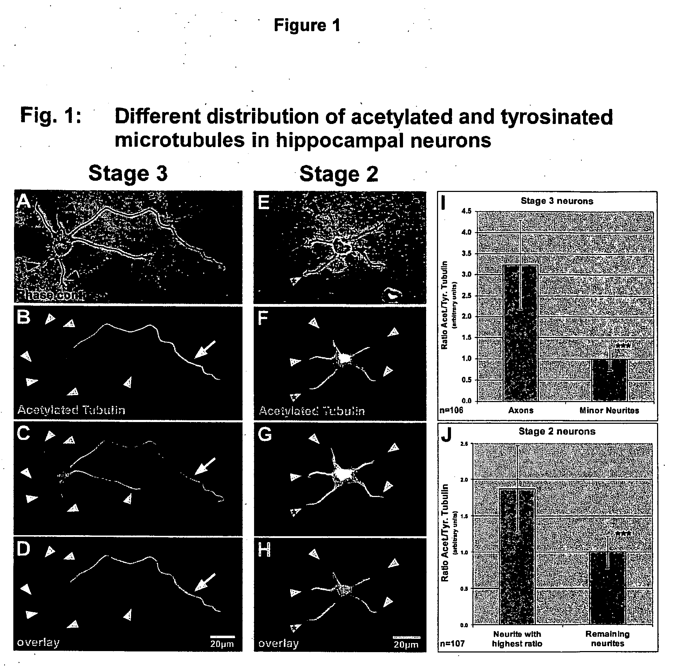

### Figure 1: Different distribution of acetylated and tyrosinated microtubules in hippocampal neurons

**(A-H)** Stage 3 (A-D) and stage 2 (E-H) rat hippocampal neurons were fixed and stained with antibodies recognizing acetylated (B, F) and tyrosinated (C,G) α-Tubulin. In stage 3 neurons, an enrichment of acetylated α-Tubulin is found in microtubules in the axon (arrow) in comparison to microtubules of minor neurites (arrowheads) (D and I). In ~50% of stage 2 neurons, the ratio acetylated/tyrosinated α-Tubulin is significantly increased in one of the minor neurites (white arrowhead with asterisk) (H and J).
(I, J) Ratio quantitation of fluorescence intensities of acetylated and tyrosinated α-Tubulin in microtubules of stage 2 (J) and stage 3 (I) neurons. In the medial part of each process, the average fluorescence intensity of both acetylated and tyrosinated α-Tubulin was measured in a square of 5x5 pixel of pictures taken with 63x magnification and the ratio of the values was calculated.

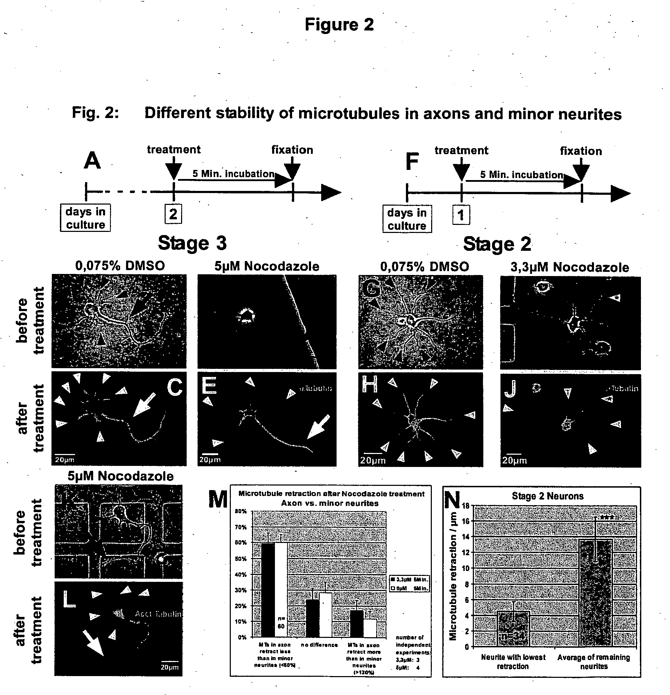

### Figure 2: Stability difference of microtubules in axons and minor neurites

Stage 3 (B-E) and stage 2 (G-J) rat hippocampal neurons were treated with 0.075% DMSO (B,C; G,H) or Nocodazole (3.3 or 5µM) (D,E; I,J) for 5 min, fixed and immunostained with an anti-α-Tubulin antibody and Rhodamine-coupled Phalloidin. In DMSO-treated control cells, microtubules reach close to the distal end (defined by the actin cytoskeleton) of axon (arrow) and minor neurites (arrowheads) (C, H). In Nocodazole-treated cells, microtubules retract towards the cell body (E, J). Retraction of axonal microtubules is significantly lower than retraction of microtubules in minor neurites (E, M). In a subpopulation of stage 2 neurons one minor neurite (arrowhead with asterisk) shows enhanced resistance to disruption by Nocodazole (J).
**(K, L)** Microtubules containing acetylated α-Tubulin show increased resistance to Nocodazole-induced depolymerization.
**(M, N)** Quantitation of the microtubule retraction of stage 3 (M) and stage 2 (N) neurons.

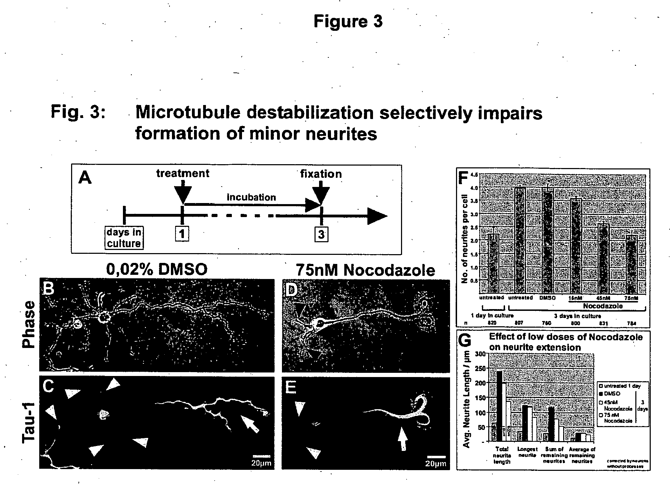

### Figure 3: Microtubule destabilization selectively impairs outgrowth of minor neurites

**(A)** After 1 DIV, low concentrations of Nocodazole or DMSO were added to the culture medium of rat hippocampal neurons. After 2 further DIV, neurons were fixed and stained for the axonal marker Tau-1.
**(B, C)** After 3 DIV, neurons have formed one axon (black arrow) and several minor neurites (black arrowheads) under control conditions (B). The axon is positive for the axonal marker Tau-1 (white arrow) and shows the typical Tau-1 gradient towards the distal part of the axon, while minor neurites are Tau-1-negative (white arrowheads) (C).
**(D, E)** While the number of minor neurites is reduced under growth conditions which slightly destabilize microtubules (D), neurons are still able to form an axon (E, white arrow).
**(F)** Nocodazole reduces the number of minor neurites formed in a concentration dependent manner.
**(G)** Neurite extension per se is not blocked by low concentrations of Nocodazole. Neurons are still growth-competent, as total neurite length increases under all growth conditions from day 1 to day 3 *in vitro.*

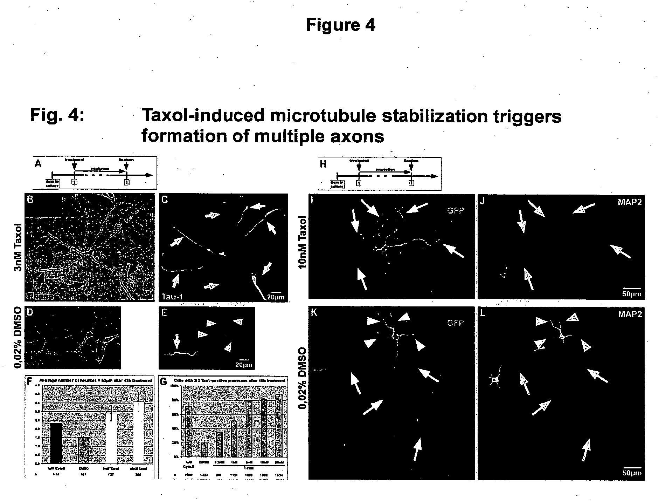

### Figure 4: Taxol-induced microtubule stabilization triggers formation of multiple axons

**(A)** After 1 DIV, rat hippocampal neurons were treated with low concentrations of Taxol or DMSO. After 2 further DIV, neurons were fixed and stained for the axonal marker Tau-1.
**(B, C)** Taxol induces the formation of multiple elongated processes (B, black arrows) which are positive for the axonal marker Tau-1 (C, white arrows).
**(D, E)** Neurons grown under control conditions have formed one Tau-1 positive axon (arrow) and several minor neurites (arrowheads).
**(F)** Effects of Taxol on neurite extension: The number of neurites longer than 60µm is increased after 2 days of Taxol-treatment, even exceeding the effect of the positive control Cytochalasin D, a drug also known to induce multiple axons (Bradke and Dotti, 1999).
**(G)** The number of cells with more than one Tau-1-positive process increases in a concentration-dependent manner in Taxol-treated neurons.
**(H)** In later stages of neuronal development cells form a dense network which makes it very hard to assign processes to the cell they originate from. To circumvent this problem and follow individual neurons, a mouse hippocampal neuron culture containing 5% of neurons which express green fluorescent protein (GFP) under the control of the Actin-promoter was used for long term experiments. The mixed culture was treated with 10nM Taxol or DMSO after 1 DIV, incubated till day 7 *in vitro,* fixed and immunostained for the dendritic marker MAP2.
**(I, J)** Taxol induces the formation of multiple long processes (I) which have no or a weak MAP2-signal (J) resembling the axons of control cells (compare L).
**(K, L)** The axon of cells grown under control conditions is MAP2-negative, the dendrites have a strong MAP2-signal.

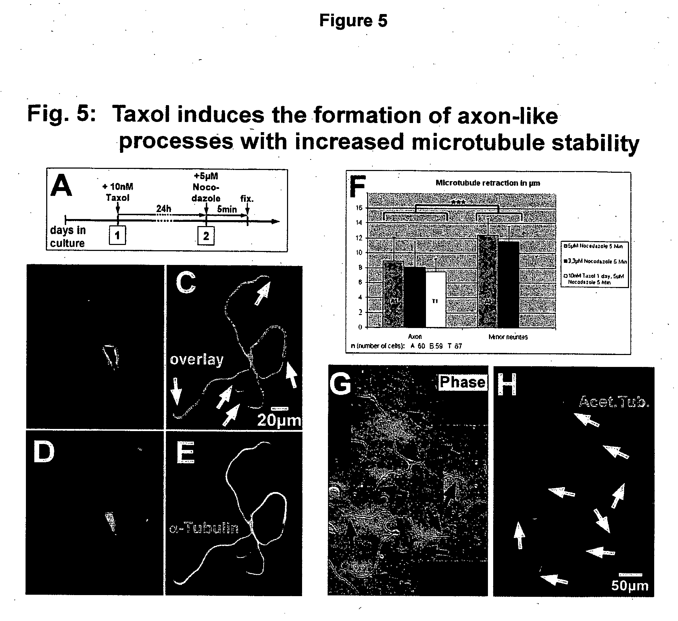

### Figure 5: Taxol induces the formation of axon-like processes with increased microtubule stability

**(A)** Stage 2 rat hippocampal neurons were treated with 10nM Taxol after 1 DIV. **(B-H)** On day 2 *in vitro,* cells had formed multiple axon-like processes (B, G, black arrows) and were treated with 0.02% DMSO (H) or 5µM Nocodazole (C-E) for 5 min, PHEM-fixed and immunostained with an anti-α-Tubulin antibody (C) and Rhodamine-coupled Phalloidin (D) or antibodies recognizing acetylated and tyrosinated α-Tubulin (H).
**(C)** In neurons pretreated with 10nM Taxol for 1 day, only little microtubule retraction occurred (C, white arrows).
**(F)** Retraction of microtubules in Taxol-treated neurons is significantly lower than retraction of microtubules in minor neurites (p<0.001) but does not differ significantly from retraction of axonal microtubules (p=0.11).
**(G, H)** Taxol-induced processes (G, black arrows) show an increased ratio of acetylated to tyrosinated alpha-Tubulin like axons (H, white arrows; compare Figure 1 D).

The invention will now be described by reference to the following biological examples which are merely illustrative and are not to be construed as a limitation of scope of the present invention.

### Example 1: Procedures and Material

### Cell Culture

Primary hippocampal neurons derived from rat embryos were cultured following the protocol of Goslin and Banker (1991) and de Hoop et al. (1997). In brief, the hippocampi of E18 rats were dissected, trypsinized, and physically dissociated. The cells were then washed in HBSS, and 1.0-1.3x10⁵ cells were plated onto polylysine-treated glass coverslips in 6 cm petri dishes containing minimal essential medium (MEM) and 10% heat-inactivated horse serum. The cells were kept in 5% CO₂ at 36.5°C. After 12-18 hr, the coverslips were transferred to a 6 cm dish containing astrocytes in MEM and N2 supplements.

### Handling Living Cells on the Microscope Stage

Neurons were kept alive and observed at the microscope as described in detail in Bradke and Dotti (1997). For short observation times, we used glass bottom dishes (MatTek Corporation, Ashland, MA) filled with 3ml HEPES-buffered HBSS, which permitted the fitting of a 12mm Cellocate coverslip (Eppendorf, Hamburg, Germany) and allowed observation with 32x and 40x objectives. Cells were kept at 36°C on the microscope stage by heating the room to an adequate temperature. Cells were illuminated with a 100 W, 12 V halogen light, which was set to minimal intensity to avoid phototoxicity.

### Videomicroscopy

Living cells were analyzed using a Zeiss Axiovert 135. The microscope was equipped with LD A-Plan 32x (NA 0.4), Plan-Apochromat 40x (NA 1.0) and Plan-Apochromat 63x (NA 1.4) objectives. Images were captured using a camera from the 4912 series (Cohu, San Diego, CA). The camera was connected to a Hamamatsu CCD camera C 2741 control panel. Pictures were recorded on the hard disc of a personal computer equipped with an image grabber (LG3 image grabber, Scion, Frederick, MD).
Cells grown on Cellocate coverslips (Eppendorf, Hamburg, Germany) were transferred into a petri dish filled with prewarmed HEPES-buffered HBSS and fixed onto a microscope stage. Cells were localized on the coverslip grid using a 32x long-distance working lens or a 40x oil immersion objective.

### Drug treatment

For long term incubation, 0.1 to 30nM Taxol (Sigma), 0.1 to 1 µM cytochalasin D (Sigma) or 3nM to 10µM Nocodazole (Sigma) were added to culture medium after 1 day in culture, and cells were further incubated at 36.5°C. Drugs were kept as stock solutions in DMSO at -20°C (Taxol: SmM; Cytochalasin D: 10mM; Nocodazole: 6,67mM).
Analyzing short term microtubule depolymerization, the fate of individual neurons was followed. Cells localized on the grid of a Cellocate coverslip were photographed. The coverslip was then transferred into HEPES-buffered HBSS containing Nocodazole and incubated for 5 min at 36.5°C in the incubator. [As Nocodazole is insoluble in aqueous solutions, special care was taken to ensure equal distribution of Nocodazole in HBSS.] After short term incubation, neurons were PHEM-fixed to eliminate depolymerized tubulin subunits and allow clear visualization of microtubules.

### Immunocytochemistry

To detect proteins by immunohistochemistry, cells were fixed in 4% paraformaldehyde for 15 min at 37°C (20 min for Tau-1 stainings), aldehyde groups were quenched in 50 mM ammonium chloride for 10 min, and the cells were then extracted with 0.1 % Triton X-100 for 3 min.
To visualize microtubules clearly, an alternative fixation method (PHEM-fixation) was used if required. To get rid of unpolymerized tubulin subunits, cells were simultaneously fixed and permeabilized for 15 min at 37°C in PHEM buffer (60 mM PIPES and 25 mM HEPES) containing 3.7% paraformaldehyde, 0.25% glutaraldehyde, 5 mM EGTA, 1mM MgCl, 3.7% sucrose, and 0.1% Triton X-100 (adapted from Smith, 1994) and quenched as above.
The neurons were then blocked at room temperature for 1 hr in a solution containing 2% fetal bovine serum (Gibco, Grand Island, NY, USA), 2% bovine serum albumin (BSA, Sigma), and 0.2% fish gelatine (Sigma) dissolved in phosphate-buffered saline. The cells were then incubated with primary antibodies diluted in 10% blocking solution. Primary antibodies used were: Tau-1 (Chemicon,
Temecula/CA, USA, 1:5.000), anti-MAP2 (Chemicon, 1:6.000), anti-αTubulin (clone B-5-1-2; Sigma, München, Germany; 1:20.000), anti-acetylated Tubulin (clone 6-11B-1; Sigma, 1:50.000), or anti-tyrosinated Tubulin YL1/2 (Abcam, Cambridge, UK, 1:40.000).
For visualization of F-Actin, Rhodamine-coupled Phalloidin (stored as a methanol stock solution at -20°C) was used (Molecular Probes, Leiden, Netherlands; 4 U/ml). As secondary antibodies, Alexa Fluor 488, 555 or 568conjugated goat anti-mouse, anti-rabbit or anti-rat IgG antibodies (Molecular probes, 1:500) were used.

### Image Analysis and Quantitation

Length and intensity measurements were done using Scion Image Beta 4.0.2 for MS Windows (based on NIH-image). For length measurements, cVisTec Professional 1.0 (cVisTec, Munich, Germany) was additionally used.
To determine the ratio of acetylated to tyrosinated α-Tubulin, the fluorescence signals of different channels of pictures taken with 63x magnification were compared. In the medial part of each process, the average fluorescence intensity of both acetylated and tyrosinated α-Tubulin was measured in a square of 5x5 pixel and the ratio of the values was determined. For minor neurites of the same cell, the average of the ratios was calculated in polarized neurons and used for comparison to the ratio of the corresponding axon. For unpolarized neurons, the highest ratio was compared to the average of the ratios of the remaining neurites. To measure microtubule retraction after Nocodazole treatment, fixed and stained neurons were relocated on the Cellocate coverslips. Pictures were taken of both the actin cytoskeleton (visualized by staining with Rhodamine-coupled Phalloidin) and the microtubules (stained with an antibody recognizing α-Tubulin). The actin cytoskeleton outlining the neuron was used as a reference point to measure retraction of microtubules from the distal part of the processes towards the cell body.
Pictures were processed using Adobe Photoshop 7.0, Deneba Canvas 8.0, and Scion Image Beta 4.0.2.

### Example 2: Different distribution of acetylated and tyrosinated microtubules in hippocampal neurons

Microtubules, along with the actin cytoskeleton and intermediate filaments one of the major components of the cellular cytoskeleton, are formed by polymerization of heterodimers of α- and β-Tubulin. Numerous studies have revealed interactions between these different components of cytoskeletal architecture (for review see 28-31). While intermediate filaments are predominantly involved in the formation of stable structures, the actin and microtubule cytoskeletons have additional roles in highly dynamic processes including cell migration, nuclear movement, cell division and cell polarity (for review see 31, 32). With the importance of the actin cytoskeleton in neuronal polarization becoming more and more evident (for review see 33), we hypothesized a role for microtubules in this complex process as well. As a first means to study a putative role of microtubules in initial neuronal polarization, we examined the distribution of different posttranslational modifications of α-Tubulin in axons and minor neurites, two types of neuronal processes in early developmental stages.
Newly synthesized α-Tubulin carries a C-terminal tyrosine residue, however, when integrated into microtubules removal of this residue from α-Tubulin occurs (34, 35). Tyrosinated α-Tubulin found in microtubules is therefore usually associated with recent assembly and serves as a marker for dynamic microtubules (36). In contrast, stable microtubular structures carry a different posttranslational modification. α-Tubulin present in such microtubules tends to be acetylated. Acetylation hence serves as a marker for stable microtubules (37).
To assess the distribution of these different posttranslational modifications in hippocampal neurons, we fixed stage 3 neurons [in 4% PFA/Sucrose] and stained for acetylated and tyrosinated α-Tubulin. Further analysis was performed using fluorescence microscopy and CCD imaging.
By means of a double band pass filter we observed an enrichment of acetylated α-Tubulin in axonal microtubules in comparison to microtubules in minor neurites in 83.5% (±1.0%; n=709) of stage 3 neurons (2 days *in vitro* DIV) (Figure 1A-D).
In contrast, tyrosinated α-Tubulin was predominant in growth cones of all processes, reflecting their dynamic state required for steering (38) and extension.
For a more quantitative analysis, we compared the ratio of the fluorescence intensities of acetylated and tyrosinated α-Tubulin in axons and minor neurites. In the axonal shaft, the ratio was increased 3.24-fold (±0.8; n=106) compared to minor neurites (Figure 1H).
To examine whether such differences already occur in unpolarized neurons, we fixed hippocampal neurons after 1 DIV and performed a similar assessment. In 54.2% (±7.8%; n=107) of stage 2 neurons, the ratio of acetylated to tyrosinated α-Tubulin of the minor neurite with the highest ratio was significantly different from the remaining minor neurites (p-value<0.01), on average the ratio was increased 1.87-fold (±0.59) in the minor neurite with the highest ratio (p-value<0.001) (Figure 1l).
Thus the axon of stage 3 neurons and one minor neurite of approximately 50% of stage 2 neurons show the typical markers of lower microtubule turnover.

### Example 3: Stability difference of microtubules in axons and minor neurites

Our next step was to address whether the different distribution of posttranslational modifications reflects an actual stability difference of microtubules in axons and minor neurites.
To test the resistance of microtubules to depolymerization, we applied the drug Nocodazole to stage 3 neurons, followed by fixation [in 3.7% PFA/Sucrose, 0.25% Glutaraldehyde, 1 x PHEM-buffer and 0.1% Triton X-100] after a brief incubation (Figure 2 A). Nocodazole is known to disrupt microtubules by binding to β-Tubulin and preventing formation of one of the two interchain disulfide linkages, thus inhibiting microtubule dynamics. After fixation, neurons were doublestained for F-Actin and α-Tubulin or acetylated and tyrosinated α-Tubulin. Fluorescence microscopy and CCD imaging was used to quantify retraction of microtubules from the distal end of processes or distribution of posttranslational modifications of α-Tubulin after partial depolymerization of the microtubule cytoskeleton.
In control cells (Figure 2 B, C) treated with DMSO microtubules reached close to the distal end of processes (Figure 2 C). However, in cells briefly treated with Nocodazole (Figure 2 D, E), we observed a retraction of microtubules towards the cell body (Figure 2 E) which was significantly higher in minor neurites compared to axons (p-value <0.001) in 60% of the cells (59.3±6.9% and 60%±5.1%, n=59 and 60, for 3.3 and 5µM Nocodazole, respectively) (Figure 2M). Thus, axonal microtubules obviously show a higher resistance to depolymerization than microtubules in minor neurites.
To scrutinize whether such a microtubule stabilization possibly precedes axon formation, we fixed hippocampal neurons after 1 DIV and performed a similar analysis. In control cells (Figure 2 G, H) treated with DMSO, microtubules reached close to the distal end of all minor neurites (Figure 2 H). In contrast, in neurons briefly treated with 3.3µM Nocodazole, the difference between microtubule retraction in the minor neurite with the lowest retraction and the average retraction of the remaining minor neurites was highly significant (4.50±1.27µm and 13.71±2.84µm, respectively; n=34; p<0.001) (Figure 2 N). Obviously the microtubules of one minor neurite show increased stability in otherwise morphologically unpolarized neurons.
In line with the distribution of stability markers in untreated stage 3 neurons (Figure 1 D) these results point to an increased stability of axonal microtubules and a possible role for microtubule stability in initial neuronal polarization.

### Example 4: Microtubule destabilization selectively impairs outgrowth of minor neurites

So far we had shown that axonal microtubules have a higher ability to resist depolymerization under harsh microtubule-destabilizing conditions. If there was a putative stability difference in axons and minor neurites, we hypothesized that particular growth conditions should allow formation of axons but not minor neurites.
To assess the effect of modest microtubule destabilization on process formation, we treated hippocampal neurons after 1 DIV with low concentrations of Nocodazole. After 2 further DIV under such growth conditions cells were fixed [with 4% PFA/Sucrose] and stained for the axonal marker Tau-1. Using fluorescence microscopy, we evaluated effects on the formation of axons and minor neurites.
In control cells, the number of processes almost doubled from day 1 to day 3 *in vitro* (2.22±0.24, 3.96±0.11, and 3.99±0.14 processes per cell after 1 DIV untreated, 3 DIV untreated or 3 DIV DMSO-treated; n=820, 807, and 760 respectively) (Figure 3F), reflecting the formation of minor neurites and an axon (Figure 3B and C). In contrast, in cells grown in the presence of low concentration of the microtubule destabilizing drug Nocodazole the number of formed processes decreased in a concentration-dependent manner (Figure 3F). Cells were still able to form an axon, however, the number of minor neurites was reduced (3.58±0.08, 2.64±0.08, and 2.20±0.18; n=800, 831 and 784 for 15, 45 and 75nM Nocodazole, respectively) (Figure 3D and E). This reduction in the number of processes was not due to a general inhibition of neurite outgrowth by Nocodazole. Total neurite length increased 4-fold from day 1 to day 3 *in vitro* under control conditions (62.7µm to 238.91µm, n=129 and 177, respectively). When treated with 45 or 75nM Nocodazole, total neurite length still increased more than 3- or 2-fold, respectively (197.31µm and 134.89µm; n=191 and 228 for 45 or 75nM Nocodazole, respectively) (Figure 3G). The clear increase of total neurite length under all conditions shows that neurons were still in a growth competent state, even when subject to slight destabilization of microtubules.
Thus, microtubule destabilization selectively impairs formation of minor neurites in early neuronal development *in vitro.*

### Example 5: Taxol-induced microtubule stabilization triggers formation of multiple axons

So far, our results pointed to an increased stability of microtubules during development and maintenance of the axon in early developmental stages.
Therefore we next wanted to asses whether microtubule stabilization itself is sufficient to induce axon formation.
To test the influence of microtubule stabilization on axon formation we treated hippocampal neurons after 1 DIV with low concentrations of the microtubule-stabilizing drug Taxol (Figure 4A and H). Taxol binds to the N-terminal region of β-Tubulin and promotes the formation of highly stable microtubules (39, 40). Taxol (also known as Paclitaxel) is successfully used as an anti-cancer drug so far as it arrests dividing cells in the G2-M-phase of cell division. Neurons grown in the presence of low concentrations of Taxol were fixed [with 4% PFA/sucrose] after 3 to 7 DIV and stained for the axonal marker Tau-1 or the dendritic marker MAP2.
Under control conditions, neurons had formed one axon and several minor neurites (Figure 4D and E). However, Taxol-treated neurons formed several elongated processes which were positive for the axonal marker Tau-1 (Figure 4B and C) and showed an increased ratio of acetylated to tyrosinated α-Tubulin like axons (Figure 5G and H, compare Figure 1D). The number of processes which exceeded 60µm had increased more than 2-fold by Taxol treatment (DMSO 1.53±0.05, 3nM Taxol 2.93±0.46, 10nM Taxol 3.58±0.54) (Figure 4F). Similarly, the number of cells with more than one Tau-1-positive process went up about 4-fold in a concentration-dependent manner in Taxol-treated neurons (from 20.19±3.19% in DMSO-treated neurons to 80.81±1.71% neurons treated with 10nM Taxol) (Figure 4G). In addition to this, Taxol-induced processes had a weak MAP2-signal resembling characteristics of axons in control neurons (Figure 4 I/J and K/L).

### Example 6: Taxol induces the formation of axon-like processes with increased microtubule stability

To test whether the low Taxol concentrations used were actually able to stabilize microtubules, we treated hippocampal neurons (1 DIV) with 10nM Taxol and performed the microtubule stability assay as described before (see 2.) after 24h incubation (Figure 5A). After brief incubation with 5µM Nocodazole, axonal microtubules had retracted on average 8.89µm (±3.23)µm; n=60) which was significantly different from the retraction of minor neurites (12.47±2.22µm; p-value<0.001). In contrast, in Taxol-induced axon-like processes little microtubule retraction occurred (Figure 5B to E) (7.48±1.76µm; n=67), which did not differ significantly from axons (p-value=0.11) (Figure 5F). Thus, stability of microtubules present in Taxol-induced processes was increased in comparison to microtubules of minor neurites and resembled the stability of axonal microtubules.

As Taxol-induced processes show typical axonal characteristics -increased length and microtubule stability, Tau-1-positive, MAP2-negative- we concluded that Taxol triggers the formation of multiple axons in hippocampal neurons.

### Example 7: Role of microtubule stabilization in mediating axonal growth in an inhibitory environment

The *in vitro* setup consists on primary culture of cerebellar granule neurons (CGN) obtained from the granular layer of the cerebellum of P7-day-old rats. In culture, these cells exhibit, over a period of 5 stages, a specific pattern of polarity development. They display a first unipolar stage with just one extended axon followed by a bipolar morphology. Then one of the emerged neurites extends two distal ramifications leading to the "T-shaped" axon morphology observed *in situ.* Cells finally become multipolar with the development of short and thick dendrites around the cell body²⁰.

This example refers to the unipolar stage and addresses the effect of microtubule stabilization on the axonal growth capacity on permissive and non-permissive substrates. CNS-myelin contains inhibitory factors which contribute to the lack of regeneration²². This inhibitory environment is reproduced by plating the cultured cells on CNS-myelin according to the protocol routinely used in laboratory¹⁶. On the basis of morphological and molecular criteria it is determined whether the growth processes of cerebellar granule neurons (CGN) is influenced by microtubule stabilization. To this end, P7 rats CGN are dissected, dissociated and plated on myelin in the presence or absence of the microtubule stabilizing drugs, including taxol. The cells are exposed to taxol (concentration range 0.1pM - 100µM) for various time periods⁷. Concomitantly, a set of cells plated and treated with C3-exoenzyme serves as growth enhancing reference ²¹. The C3-exoenzyme is commercially available. After 36 hours of culture, the cells are fixed and stained with a neuron-specific beta tubulin antibody. Pictures are made from the neurons and axonal length is measured using the Scion Image software (NIH, Bethesda, USA).

### Example 8: Effects of microtubulule stabilization with taxol on anatomical and functional outcomes

This example describes the assessment of whether a microtubule stablizing compound such as taxol promotes axonal growth *in vivo* and improves the clinical outcome of spinal cord injury. The injury is induced by a dorsal hemi-section of the spinal cord at thoracic level (T8)²³. This bilateral hemi-section interrupts the multiple motor control projections and so leads to a diminution of the locomotor performances. Of particular interest is the corticospinal tract (CST) and the raphaespinal tract. The interruption of these tracts depresses the locomotor function¹² (and Saruhashi et al., 1996; Antri et al., 2002; Kim et al., 2004 and Lee et al., 2004).

First, the lesion site is characterized in the used spinal cord injury paradigm. The consequences of the hemi-section on the spinal tracts and on the locomotor's functions are also evaluated. Then it is examined whether taxol induces change in the lesion site, axonal outgrowth and eventually improves the functional recovery.

Taxol or its vehicle is continuously and locally delivered at the site of injury via a catheter connected to an osmotic minipump (2004, Alzet) and inserted into the intrathecal space of the spinal cord. To determine the optimal dose for the administration of treatment, first taxol is perfused at concentrations ranging from 0.01 pM to 100 µM/hour. The treatment is started one week following injury. In case the treatment interferes with scarring and causes additional damage at the lesion, then it is delayed until two weeks following injury. Delayed post-injury deliveries were chosen because it is the more significant clinical paradigm: the glial scar is already installed at these times⁴ and therefore it allows to focus on the regenerative process.
- *Lesion site.* First, a qualitative characterization of the different lesion paradigms is done. Sham-operated (laminectomy only) and spinal-injured are fixed at 2- 4 and 6 weeks post-lesion. Coronal and horizontal serial sections of the cords are carried out where histological staining is performed and the following immunohistochemical markers are analyzed.
   First sections are stained with Hematoxilin Eosin to assess the extent of the necrotic cavities and of the scar tissue around the lesion site induced by the injury. The horizontal sections are stained with Luxol Fast Blue to identify the myelinated white matter and the amount of the spared fibers at the lesion site.
   Then address the cellular response to injury is addressed by immuno-labelling the coronal sections on the adjacent slides series. Based on the change of the morphology of the cells, the astroglial and microglial reactivity are examined which will reflect the occurring inflammatory process using glial fibrillary acidic protein (GFAP) and OX42 antibody respectively⁴. Additionally, it is evaluated if the model leads to the proliferation of oligodendrocyte progenitors by testing 04 marker immunoreactivity (Ishii et al., 2001).
   To test whether taxol causes changes at the lesion site, the effect of taxol on the lesion paradigms defined above is studied.
- *Axonal growth.* The effect of taxol on axonal growth is evaluated by labelling the CST and the Raphae spinal tract. This technique is first applied in spinal cord-injured and non-injured rats to control the efficiency of the transection induced by the hemi-section. Then the effect on taxol on the transected neurons is evaluated. To study the growth velocity of axonal growth in detail, the animals are analysed after different time points ranging from 3 days to 6 weeks post-treatment.
   Tthe transected neurons of the CST are labelled with the anterograde tracer biotinylated dextran amine (BDA). This anterograde tracer permits to completely reconstruct the axonal arbors of the CST (Bareyre et al., 2004). The CST is running from the sensorimotor areas of the cortex through the brainstem to the spinal cord and is involved in the voluntary motor functions (Smith and Bennett, 1987; Tracey (1995)). It can be anterogradely labelled and in murine animals, the majority of its fibers are localized in the ventral part the dorsal column, dorsal to the central canal. Then this tract can be a useful tool for regeneration examination. BDA is applied bilaterally by stereotaxic injections at different sites of the sensorimotor cortex (Paxinos and Watson, 1982). After 2 weeks, the time required for the tracer to be transported along the CST pathway¹³ (and Lee et al., 2004; Reiner et al., 2000), the rats are perfused with fixative. The spinal cords are dissected and cut into 2 cm lengths. BDA stain is then visualized using the avidin-biotinylated HRP procedure on serial horizontal sections (Reiner et al., 2000).
   The anatomic part of the study is completed by examining the effect of taxol on the Raphe nuclei projections into the spinal cord with serotonin immuno-labelling. The raphaespinal tract as well as the CST strongly participated to the locomotor functions (Schmidt and Jordan, 2000). Its fibers can be also easily visualized through the spinal cord by the serotonin immuno-labelling. At the thoracic level a dense network of serotonin immuno-reactivity is present in the spinal cord dorsolateral funiculus which is partially affected by the hemi-section (Paxinos and Watson, 1982; Schmidt and Jordan, 2000).
- *Functional recovery,* Effect of taxol on functional recovery are examined by measuring the hindlimb performances weekly for the length of the study using the Basso - Beattie - Bresnahan (BBB) locomotor rating scale.
   The locomotor deficit is a first estimate on control injured compared to sham animals. To test whether taxol treatment contributes to a functional improvement, compare the locomotor behaviour of the vehicle- and taxol-treated animals is compared.
   The spinal hemi-section performed at the thoracic level dramatically impaired the hindlimb functions leading to a complete hindlimb paralysis during the first days post-injury. Afterwards, the hindlimb performances gradually improve. Therefore, locomotion constitutes a clinical relevant behaviour to predict neurological recovery. The BBB scale which covers a wide range of motor deficits is accurately designed to assess qualitative hindlimbs recovery after spinal cord injury. The animals of each group are carefully handled and placed on the open-field and observed during 5 minutes. From the observation of the articulation movement of the 3 joints, (hip, knee and ankle) an individual score for each leg is attributed from 0 (paralysis) to 21 (normal walking). The components of the hindlimb movement include weight support, plantar and dorsal stepping, forelimb-hindlimb coordination, paw rotation, toe clearance, trunk stability and tail placement. The BBB scale defines 3 level of recovery. The first level (between 0 and 8) describes the early phase of recovery by scoring the small or large movements of the 3 joints of the hindlimb. Scores from 9 to 13 defines the intermediate phase of recovery focused on weight support capacity. The last level (from 14 to 21) indicates the progressive improvements in the coordinated walking ability (Basso et al., 1995).

### Abbreviations:

- **DIV**: days *in vitro*
- **PFA**: Paraformaldehyde

### References:

1 Qiu J. and Filbin M.T. (2000). Glia 29,166-174.
2 Sandvig A., Berry M., Barrett L.B., Butt A., Logan A. (2004). Glia 46, 225-251.
3 Li M., Shibata A., Li C., Braun P.E., McKerracher L., Roder J., Kater S.B., David S. (1996). J Neurosci Res. 46, 404-414.
4 Silver J. and Miller J. H. (2004). Nat Rev Neurosci 5, 146-156.
5 Luo L. (2000). Nat Rev Neurosci 1, 173-180.
6 Dent E.W., Gertler F.B. (2003). Neuron 40, 209-227.
7 Bradke F. and Dotti C. G. (1999). Science 283, 1931-1934.
8 Winton MJ, Dubreuil Cl, Lasko D, Leclerc N, McKerracher L. (2002). J Biol Chem. 277, 32820-32829.
9 Wilde C., Vogelsgesang M., Aktories K. (2003). Biochemistry. 42, 9694-9702.
10 Lehmann M., Fournier A., Selles-Navarro I., Dergham P., Sebok A., Leclerc N., Tigyi G., McKerracher L. (1999). J Neurosci. 19, 7537-7547.
11 Borisoff J. F., Chan C. C., Hiebert G. W., Oschipok L., Robertson G. S., Zamboni R., Steeves J. D., Tetzlaff W. (2003). Mol Cell Neurosci. 22, 405-416.
12 Dergham P, Ellezam B, Essagian C, Avedissian H, Lubell WD, McKerracher L. (2002). J Neurosci. 22, 6570-6577.
13 Fournier A.E., Takizawa B.T., Strittmatter S.M. (2003). J Neurosci. 23, 1416-1423.
14 Etienne-Manneville S. and Hall A. (2002). Nature 420, 629-635.
15 Wettschureck N., Offermanns S., (2002). J Mol Med. 80, 629-638
16 Neumann S., Bradke F., Tessier-Lavigne M., Basbaum A. I. (2002). Neuron 34, 885-893.
17 Bradke F. and Dotti C. G. (2000). Curr Biol 10, 1467-1470.
21 Bito H., Furuyashiki T., Ishihara H., Shibasaki Y., Ohashi K., Mizuno K., Maekawa M., Ishizaki T., Narumiya S. (2000). Neuron 26, 431-441.
22 Caroni P. and Schwab M. E. (1988). J Cell Biol 106, 1281-1288.
23 Neumann S. and Woolf C. J. (1999). Neuron 23, 83-91.
24 Dotti, C et al., (1988). J. Neurosci. 8:1454-68.
25 Dotti, CG, and Banker (1987). Nature 330:254-6.
26 Bradke, F., and Dotti, C. G. (1999). The role of local actin instability in axon formation. Science 283, 1931-1934
27 Smith, C. L. (1994). Cytoskeletal movements and substrate interactions during initiation of neurite outgrowth by sympathetic neurons in vitro. J Neurosci 14, 384-398
28 Gordon-Weeks, P. R. Microtubules and growth cone function. J Neurobiol 58, 70-83 (2004).
29 Schliwa, M. & Honer, B. Microtubules, centrosomes and intermediate filaments in directed cell movement. Trends Cell Biol 3, 377-80 (1993).
30 Yarm, F., Sagot, I. & Pellman, D. The social life of actin and microtubules: interaction versus cooperation. Curr Opin Microbiol 4, 696-702 (2001).
31 Rodriguez, O. C. et al. Conserved microtubule-actin interactions in cell movement and morphogenesis. Nat Cell Biol 5, 599-609 (2003).
32 Etienne-Manneville, S. Actin and microtubules in cell motility: which one is in control? Traffic 5, 470-7 (2004).
33 da Silva, J. S. & Dotti, C. G. Breaking the neuronal sphere: regulation of the actin cytoskeleton in neuritogenesis. Nat Rev Neurosci 3, 694-704 (2002).
34. Argarana, C. E., Barra, H. S. & Caputto, R. Release of [14C]tyrosine from tubulinyl-[14C]tyrosine by brain extract. Separation of a carboxypeptidase from tubulin-tyrosine ligase. Mol Cell Biochem 19, 17-21 (1978).
35. Wehland, J. & Weber, K. Turnover of the carboxy-terminal tyrosine of alpha-tubulin and means of reaching elevated levels of detyrosination in living cells. J Cell Sci 88 ( Pt 2), 185-203 (1987).
36. Webster, D. R., Gundersen, G. G., Bulinski, J. C. & Borisy, G. G. Differential turnover of tyrosinated and detyrosinated microtubules. Proc Natl Acad Sci U S A 84, 9040-4 (1987).
37. Sasse, R. & Gull, K. Tubulin post-translational modifications and the construction of microtubular organelles in Trypanosoma brucei. J Cell Sci 90 (Pt 4), 577-89 (1988).
38. Buck, K. B. & Zheng, J. Q. Growth cone turning induced by direct local modification of microtubule dynamics. J Neurosci 22, 9358-67 (2002).
39. Schiff, P. B., Fant, J. & Horwitz, S. B. Promotion of microtubule assembly in vitro by taxol. Nature 277, 665-7 (1979).
40. Schiff, P. B. & Horwitz, S. B. Taxol stabilizes microtubules in mouse fibroblast cells. Proc Natl Acad Sci U S A 77, 1561-5 (1980)
41. Perez-Espejo MA, Haghighi SS, Adelstein EH, Madsen R. The effects of taxol, methylprednisolone, and 4-aminopyridine in compressive spinal cord injury: a qualitative experimental study. Surg Neurol.;46(4):350-7 (1996).
42. National Cancer Institute, Division of Cancer Treatment; Taxol. IND 22850. NSC 125973. Clinical brochure. Bethesda: 26-27 (1992).
43. Mercado-Gomez O, Ferrera P, Arias C. Histopathologic changes induced by the microtubule-stabilizing agent Taxol in the rat hippocampus in vivo. J Neurosci Res. 78(4):553-62 (2004).
44. Smith DS and Skene JH; A transcription-dependent switch controls competence of adult neurons for distinct modes of axon growth. J Neurosci. 17(2):646-58 (1997).

Bocca, C., Gabriel, L., Bozzo, F., and Miglietta, A. (2002). Microtubule-interacting activity and cytotoxicity of the prenylated coumarin ferulenol. Planta Med 68, 1135-1137.
Bocca, C., Gabriel, L., Bozzo, F., and Miglietta, A. (2004). A sesquiterpene lactone, costunolide, interacts with microtubule protein and inhibits the growth of MCF-7 cells. Chem Biol Interact 147, 79-86.
Buck, K. B., and Zheng, J. Q. (2002). Growth cone turning induced by direct local modification of microtubule dynamics. J Neurosci 22, 9358-9367.
Cinel, B., Roberge, M., Behrisch, H., van Ofwegen, L., Castro, C. B., and Andersen, R. J. (2000). Antimitotic diterpenes from Erythropodium caribaeorum test pharmacophore models for microtubule stabilization. Org Lett 2, 257-260.
David, B., Sevenet, T., Morgat, M., Guenard, G., Moisand, A., Tollon, Y., Thoison, O., and Wright, M. (1994). Rhazinilam mimics the cellular effects of taxol by different mechanisms of action. Cell Motil Cytoskeleton 28, 317-326.
Dietzmann, A., Kanakis, D., Kirches, E., Kropf, S., Mawrin, C., and Dietzmann, K. (2003). Nanomolar concentrations of epothilone D inhibit the proliferation of glioma cells and severely affect their tubulin cytoskeleton. J Neurooncol 65, 99-106.
Hamel, E., Sackett, D. L., Vourloumis, D., and Nicolaou, K. C. (1999). The coral-derived natural products eleutherobin and sarcodictyins A and B: effects on the assembly of purified tubulin with and without microtubule-associated proteins and binding at the polymer taxoid site. Biochemistry 38, 5490-5498.
Kavallaris, M., Verrills, N. M., and Hill, B. T. (2001). Anticancer therapy with novel tubulin-interacting drugs. Drug Resist Updat 4, 392-401.
Klar, U., Graf, H., Schenk, O., Rohr, B., and Schulz, H. (1998). New synthetic inhibitors of microtubule depolymerization. Bioorg Med Chem Lett 8, 1397-1402.
Long, B. H., Carboni, J. M., Wasserman, A. J., Cornell, L. A., Casazza, A. M., Jensen, P. R., Lindel, T., Fenical, W., and Fairchild, C. R. (1998). Eleutherobin, a novel cytotoxic agent that induces tubulin polymerization, is similar to paclitaxel (Taxol). Cancer Res 58, 1111-1115.
Madari, H., Panda, D., Wilson, L., and Jacobs, R. S. (2003). Dicoumarol: a unique microtubule stabilizing natural product that is synergistic with Taxol. Cancer Res 63, 1214-1220.
Miglietta, A., Bozzo, F., Gabriel, L., and Bocca, C. (2004). Microtubule-interfering activity of parthenolide. Chem Biol Interact 149, 165-173.
Mooberry, S. L., Stratman, K., and Moore, R. E. (1995). Tubercidin stabilizes microtubules against vinblastine-induced depolymerization, a taxol-like effect. Cancer Lett 96, 261-266.
Nakamura, M., Nakazawa, J., Usui, T., Osada, H., Kono, Y., and Takatsuki, A. (2003). Nordihydroguaiaretic acid, of a new family of microtubule-stabilizing agents, shows effects differentiated from paclitaxel. Biosci Biotechnol Biochem 67, 151-157.
Nicolaou, K. C., Riemer, C., Kerr, M. A., Rideout, D., and Wrasidlo, W. (1993). Design, synthesis and biological activity of protaxols. Nature 364, 464-466.
Ojima, I., Chakravarty, S., Inoue, T., Lin, S., He, L., Horwitz, S. B., Kuduk, S. D., and Danishefsky, S. J. (1999). A common pharmacophore for cytotoxic natural products that stabilize microtubules. Proc Natl Acad Sci U S A 96, 4256-4261.
Rao, S., He, L., Chakravarty, S., Ojima, I., Orr, G. A., and Horwitz, S. B. (1999). Characterization of the Taxol binding site on the microtubule. Identification of Arg(282) in beta-tubulin as the site of photoincorporation of a 7-benzophenone analogue of Taxol. J Biol Chem 274, 37990-37994.
Rao, S., Krauss, N. E., Heerding, J. M., Swindell, C. S., Ringel, I., Orr, G. A., and Horwitz, S. B. (1994). 3'-(p-azidobenzamido)taxol photolabels the N-terminal 31 amino acids of beta-tubulin. J Biol Chem 269, 3132-3134.
Ringel, I., and Horwitz, S. B. (1991). Studies with RP 56976 (taxotere): a semisynthetic analogue of taxol. J Natl Cancer Inst 83, 288-291.
Shintani, Y., Tanaka, T., and Nozaki, Y. (1997). GS-164, a small synthetic compound, stimulates tubulin polymerization by a similar mechanism to that of Taxol. Cancer Chemother Pharmacol 40, 513-520.
Taraboletti, G., Micheletti, G., Rieppi, M., Poli, M., Turatto, M., Rossi, C., Borsotti, P., Roccabianca, P., Scanziani, E., Nicoletti, M. I., et al. (2002). Antiangiogenic and antitumor activity of IDN 5390, a new taxane derivative. Clin Cancer Res 8, 1182-1188.
Tinley, T. L., Randall-Hlubek, D. A., Leal, R. M., Jackson, E. M., Cessac, J. W., Quada, J. C., Jr., Hemscheidt, T. K., and Mooberry, S. L. (2003). Taccalonolides E and A: Plant-derived steroids with microtubule-stabilizing activity. Cancer Res 63, 3211-3220.
Uyar, D., Takigawa, N., Mekhail, T., Grabowski, D., Markman, M., Lee, F., Canetta, R., Peck, R., Bukowski, R., and Ganapathi, R. (2003). Apoptotic pathways of epothilone BMS 310705. Gynecol Oncol 91, 173-178.
Wu, J. H., Batist, G., and Zamir, L. O. (2001). Identification of a novel steroid derivative, NSC12983, as a paclitaxel-like tubulin assembly promoter by 3-D virtual screening. Anticancer Drug Des 16, 129-133.
Bradke, F. and Dotti, C. G. (1997) Neuronal Polarity: Vectorial Cytoplasmic Flow Precedes Axon Formation. Neuron 19, 1175-1186.
Ali, S. M., Hoemann, M. Z., Aube, J., Georg, G. I., Mitscher, L. A., and Jayasinghe, L. R. (1997). Butitaxel analogues: synthesis and structure-activity relationships. J Med Chem 40, 236-241.
Altstadt, T. J., Fairchild, C. R., Golik, J., Johnston, K. A., Kadow, J. F., Lee, F. Y., Long, B. H., Rose, W. C., Vyas, D. M., Wong, H., et al. (2001). Synthesis and antitumor activity of novel C-7 paclitaxel ethers: discovery of BMS-184476. J Med Chem 44, 4577-4583.
Bissery, M. C. (2001). Preclinical evaluation of new taxoids. Curr Pharm Des 7, 1251--1257.
Bocca, C., Gabriel, L., Bozzo, F., and Miglietta, A. (2002). Microtubule-interacting activity and cytotoxicity of the prenylated coumarin ferulenol. Planta Med 68, 1135-1137.
Bocca, C., Gabriel, L., Bozzo, F., and Miglietta, A. (2004). A sesquiterpene lactone, costunolide, interacts with microtubule protein and inhibits the growth of MCF-7 cells. Chem Biol Interact 147, 79-86.
Bollag, D. M., McQueney, P. A., Zhu, J., Hensens, O., Koupal, L., Liesch, J., Goetz, M., Lazarides, E., and Woods, C. M. (1995). Epothilones, a new class of microtubule-stabilizing agents with a taxol-like mechanism of action. Cancer Res 55, 2325-2333.
Bradley, M. O., Swindell, C. S., Anthony, F. H., Witman, P. A., Devanesan, P., Webb, N. L, Baker, S. D., Wolff, A. C., and Donehower, R. C. (2001a). Tumor targeting by conjugation of DHA to paclitaxel. J Control Release 74, 233-236.
Bradley, M. O., Webb, N. L., Anthony, F. H., Devanesan, P., Witman, P. A., Hemamalini, S., Chander, M. C., Baker, S. D., He, L., Horwitz, S. B., and Swindell, C. S. (2001 b). Tumor targeting by covalent conjugation of a natural fatty acid to paclitaxel. Clin Cancer Res 7, 3229-3238.
Buck, K. B., and Zheng, J. Q. (2002). Growth cone turning induced by direct local modification of microtubule dynamics. J Neurosci 22, 9358-9367.
Carboni, J. M., Farina, V., Rao, S., Hauck, S. I., Horwitz, S. B., and Ringel, I. (1993). Synthesis of a photoaffinity analog of taxol as an approach to identify the taxol binding site on microtubules. J Med Chem 36, 513-515.
Chou, T. C., Dong, H., Rivkin, A., Yoshimura, F., Gabarda, A. E., Cho, Y. S., Tong, W. P., and Danishefsky, S. J. (2003). Design and total synthesis of a superior family of epothilone analogues, which eliminate xenograft tumors to a nonrelapsable state. Angew Chem Int Ed Engl 42, 4762-4767.
Chou, T. C., O'Connor, O. A., Tong, W. P., Guan, Y., Zhang, Z. G., Stachel, S. J., Lee, C., and Danishefsky, S. J. (2001). The synthesis, discovery, and development of a highly promising class of microtubule stabilization agents: curative effects of desoxyepothilones B and F against human tumor xenografts in nude mice. Proc Natl Acad Sci U S A 98, 8113-8118.
Chou, T. C., Zhang, X. G., Balog, A., Su, D. S., Meng, D., Savin, K., Bertino, J. R., and Danishefsky, S. J. (1998a). Desoxyepothilone B: an efficacious microtubule-targeted antitumor agent with a promising in vivo profile relative to epothilone B. Proc Natl Acad Sci U S A 95, 9642-9647.
Chou, T. C., Zhang, X. G., Harris, C. R., Kuduk, S. D., Balog, A., Savin, K. A., Bertino, J. R., and Danishefsky, S. J. (1998b). Desoxyepothilone B is curative against human tumor xenografts that are refractory to paclitaxel. Proc Natl Acad Sci U S A 95, 15798-15802.
Cinel, B., Roberge, M., Behrisch, H., van Ofwegen, L., Castro, C. B., and Andersen, R. J. (2000). Antimitotic diterpenes from Erythropodium caribaeorum test pharmacophore models for microtubule stabilization. Org Lett 2, 257-260.
Cisternino, S., Bourasset, F., Archimbaud, Y., Semiond, D., Sanderink, G., and Scherrmann, J. M. (2003). Nonlinear accumulation in the brain of the new taxoid TXD258 following saturation of P-glycoprotein at the blood-brain barrier in mice and rats. Br J Pharmacol 138, 1367-1375.
David, B., Sevenet, T., Morgat, M., Guenard, G., Moisand, A., Tollon, Y., Thoison, O., and Wright, M. (1994). Rhazinilam mimics the cellular effects of taxol by different mechanisms of action. Cell Motil Cytoskeleton 28, 317-326.
Dietzmann, A., Kanakis, D., Kirches, E., Kropf, S., Mawrin, C., and Dietzmann, K. (2003). Nanomolar concentrations of epothilone D inhibit the proliferation of glioma cells and severely affect their tubulin cytoskeleton. J Neurooncol 65, 99-106.
Fumoleau, P., Trigo, J., Campone, M., Baselga, J., Sistac, F., Gimenez, L., and al., e. (2001). Phase I and pharmacokinetic (PK) study of RPR 116258A, given as a weekly 1-hour infusion at day 1, day 8, day 15, day 22 every 5 weeks in patients (pts) with advanced solid tumors. Proceedings of the 2001 AACR-NCI-EORTC International Conference Résumé 282, 58*.*
Goetz, A. D., Denis, L. J., Rowinsky, E. K., Ochoa, L., Mompus, K., Deblonde, B., and al., e. (2001). Phase I and pharmacokinetic study of RPR 116258A, a novel taxane derivative, administered intravenously over 1 hour every 3 weeks. Proceedings of ASCO 20, 419 (abstr.), 106a.
Goodin, S., Kane, M. P., and Rubin, E. H. (2004). Epothilones: mechanism of action and biologic activity. J Clin Oncol 22, 2015-2025.
Haggarty, S. J., Mayer, T. U., Miyamoto, D. T., Fathi, R., King, R. W., Mitchison, T. J., and Schreiber, S. L. (2000). Dissecting cellular processes using small molecules: identification of colchicine-like, taxol-like and other small molecules that perturb mitosis. Chem Biol 7, 275-286.
Hamel, E., Sackett, D. L., Vourloumis, D., and Nicolaou, K. C. (1999). The coral-derived natural products eleutherobin and sarcodictyins A and B: effects on the assembly of purified tubulin with and without microtubule-associated proteins and binding at the polymer taxoid site. Biochemistry 38, 5490-5498.
Heller, J. D., Kuo, J., Wu, T. C., Kast, W. M., and Huang, R. C. (2001). Tetra-O-methyl nordihydroguaiaretic acid induces G2 arrest in mammalian cells and exhibits tumoricidal activity in vivo. Cancer Res 61, 5499-5504.
Hood, K. A., West, L. M., Rouwe, B., Northcote, P. T., Berridge, M. V., Wakefield, S. J., and Miller, J. H. (2002). Peloruside A, a novel antimitotic agent with paclitaxel-like microtubule- stabilizing activity. Cancer Res 62, 3356-3360.
Hung, D. T., Chen, J., and Schreiber, S. L. (1996). (+)-Discodermolide binds to microtubules in stoichiometric ratio to tubulin dimers, blocks taxol binding and results in mitotic arrest. Chem Biol 3, 287-293.
limura, S., Uoto, K., Ohsuki, S., Chiba, J., Yoshino, T., lwahana, M., Jimbo, T., Terasawa, H., and Soga, T. (2001). Orally active docetaxel analogue: synthesis of 10-deoxy-10-C-morpholinoethyl docetaxel analogues. Bioorg Med Chem Lett 11, 407-410.
Kim, T. Y., Kim, D. W., Chung, J. Y., Shin, S. G., Kim, S. C., Heo, D. S., Kim, N. K., and Bang, Y. J. (2004). Phase I and pharmacokinetic study of Genexol-PM, a cremophor-free, polymeric micelle-formulated paclitaxel, in patients with advanced malignancies. Clin Cancer Res 10, 3708-3716.
Klar, U., Graf, H., Schenk, O., Rohr, B., and Schulz, H. (1998). New synthetic inhibitors of microtubule depolymerization. Bioorg Med Chem Lett 8, 1397-1402.
Kolman, A. (2004). Epothilone D (Kosan/Roche). Curr Opin Investig Drugs 5, 657-667.
Koshkina, N. V., Waldrep, J. C., Roberts, L. E., Golunski, E., Melton, S., and Knight, V. (2001). Paclitaxel liposome aerosol treatment induces inhibition of pulmonary metastases in murine renal carcinoma model. Clin Cancer Res 7, 3258-3262.
Kowalski, R. J., Giannakakou, P., Gunasekera, S. P., Longley, R. E., Day, B. W., and Hamel, E. (1997a). The microtubule-stabilizing agent discodermolide competitively inhibits the binding of paclitaxel (Taxol) to tubulin polymers, enhances tubulin nucleation reactions more potently than paclitaxel, and inhibits the growth of paclitaxel-resistant cells. Mol Pharmacol 52, 613-622.
Kowalski, R. J., Giannakakou, P., and Hamel, E. (1997b). Activities of the microtubule-stabilizing agents epothilones A and B with purified tubulin and in cells resistant to paclitaxel (Taxol(R)). J Biol Chem 272, 2534-2541.
Kurata, T., Shimada, Y., Tamura, T., Yamamoto, N., Hyodo, I., Saeki, T., Takashima, S., Fujiwara, K., Wakasugi, H., and Kashimura, M. (2000). Phase I and pharmacokinetic study of a new taxoid, RPR 109881A, given as a 1-hour intravenous infusion in patients with advanced solid tumors. J Clin Oncol 18, 3164-3171.
Langer, C. J. (2004). CT-2103: a novel macromolecular taxane with potential advantages compared with conventional taxanes. Clin Lung Cancer 6 Suppl 2, S85-88.
Leung, S. Y., Jackson, J., Miyake, H., Burt, H., and Gleave, M. E. (2000). Polymeric micellar paclitaxel phosphorylates Bcl-2 and induces apoptotic regression of androgen-independent LNCaP prostate tumors. Prostate 44, 156-163.
Li, C., Newman, R. A., Wu, Q. P., Ke, S., Chen, W., Hutto, T., Kan, Z., Brannan, M. D., Charnsangavej, C., and Wallace, S. (2000). Biodistribution of paclitaxel and poly(L-glutamic acid)-paclitaxel conjugate in mice with ovarian OCa-1 tumor. Cancer Chemother Pharmacol 46, 416-422.
Li, C., Yu, D. F., Newman, R. A., Cabral, F., Stephens, L. C., Hunter, N., Milas, L., and Wallace, S. (1998). Complete regression of well-established tumors using a novel water-soluble poly(L-glutamic acid)-paclitaxel conjugate. Cancer Res 58, 2404-2409.
Lindel, T., Jensen, P. R., Fenical, W., Long, B. H., Casazza, A. M., Carboni, J., and Fairchild, C. R. (1997). Eleutherobin, a new cytotoxin that mimics paclitaxel (Taxol) by stabilizing microtubules. J Am Chem Soc 119, 8744-8745.
Lorthoraly, A., Pierga, J. Y., Delva, R., Girre, V., Gamelin, E., Terpereau, A., and al., e. (2000). Phase I and pharmacokinetic (PK) study of RPR 116258A given as a 1-hour infusion in patients (pts) with advanced solid tumors. Proceedings of the 11th NCI-EORTC-AACR Symposium 2000 (résumé 569), 156-157.
Madari, H., Panda, D., Wilson, L., and Jacobs, R. S. (2003). Dicoumarol: a unique microtubule stabilizing natural product that is synergistic with Taxol. Cancer Res 63, 1214-1220.
Martello, L. A., McDaid, H. M., Regl, D. L., Yang, C. P., Meng, D., Pettus, T. R., Kaufman, M. D., Arimoto, H., Danishefsky, S. J., Smith, A. B., 3rd, and Horwitz, S. B. (2000). Taxol and discodermolide represent a synergistic drug combination in human carcinoma cell lines. Clin Cancer Res 6, 1978-1987.
Meerum Terwogt, J. M., ten Bokkel Huinink, W. W., Schellens, J. H., Schot, M., Mandjes, I. A., Zurlo, M. G., Rocchetti, M., Rosing, H., Koopman, F. J., and Beijnen, J. H. (2001). Phase I clinical and pharmacokinetic study of PNU166945, a novel water-soluble polymer-conjugated prodrug of paclitaxel. Anticancer Drugs 12, 315-323.
Miglietta, A., Bozzo, F., Gabriel, L., and Bocca, C. (2004). Microtubule-interfering activity of parthenolide. Chem Biol Interact 149, 165-173.
Mooberry, S. L., Stratman, K., and Moore, R. E. (1995). Tubercidin stabilizes microtubules against vinblastine-induced depolymerization, a taxol-like effect. Cancer Lett 96, 261-266.
Mooberry, S. L., Tien, G., Hernandez, A. H., Plubrukarn, A., and Davidson, B. S. (1999). Laulimalide and isolaulimalide, new paclitaxel-like microtubule-stabilizing agents. Cancer Res 59, 653-660.
Multani, A. S., Li, C., Ozen, M., Yadav, M., Yu, D. F., Wallace, S., and Pathak, S. (1997). Paclitaxel and water-soluble poly (L-glutamic acid)-paclitaxel, induce direct chromosomal abnormalities and cell death in a murine metastatic melanoma cell line. Anticancer Res 17, 4269-4274.
Nakamura, M., Nakazawa, J., Usui, T., Osada, H., Kono, Y., and Takatsuki, A. (2003). Nordihydroguaiaretic acid, of a new family of microtubule-stabilizing agents, shows effects differentiated from paclitaxel. Biosci Biotechnol Biochem 67, 151-157.
Nicolaou, K. C., Riemer, C., Kerr, M. A., Rideout, D., and Wrasidlo, W. (1993). Design, synthesis and biological activity of protaxols. Nature 364, 464-466.
Nicoletti, M. I., Colombo, T., Rossi, C., Monardo, C., Stura, S., Zucchetti, M., Riva, A., Morazzoni, P., Donati, M. B., Bombardelli, E., et al. (2000). IDN5109, a taxane with oral bioavailability and potent antitumor activity. Cancer Res 60, 842-846.
Ojima, I., Slater, J. C., Michaud, E., Kuduk, S. D., Bounaud, P. Y., Vrignaud, P., Bissery, M. C., Veith, J. M., Pera, P., and Bernacki, R. J. (1996). Syntheses and structure-activity relationships of the second-generation antitumor taxoids: exceptional activity against drug-resistant cancer cells. J Med Chem 39, 3889-3896.
Oldham, E. A., Li, C., Ke, S., Wallace, S., and Huang, P. (2000). Comparison of action of paclitaxel and poly(L-glutamic acid)-paclitaxel conjugate in human breast cancer cells. Int J Oncol 16, 125-132.
Ramaswamy, M., Zhang, X., Burt, H. M., and Wasan, K. M. (1997). Human plasma distribution of free paclitaxel and paclitaxel associated with diblock copolymers. J Pharm Sci 86, 460-464.
Rao, S., He, L., Chakravarty, S., Ojima, I., Orr, G. A., and Horwitz, S. B. (1999). Characterization of the Taxol binding site on the microtubule. Identification of Arg(282) in beta-tubulin as the site of photoincorporation of a 7-benzophenone analogue of Taxol. J Biol Chem 274, 37990-37994.
Rao, S., Krauss, N. E., Heerding, J. M., Swindell, C. S., Ringel, I., Orr, G. A., and Horwitz, S. B. (1994). 3'-(p-azidobenzamido)taxol photolabels the N-terminal 31 amino acids of beta-tubulin. J Biol Chem 269, 3132-3134.
Rao, S., Orr, G. A., Chaudhary, A. G., Kingston, D. G., and Horwitz, S. B. (1995). Characterization of the taxol binding site on the microtubule. 2-(m-Azidobenzoyl)taxol photolabels a peptide (amino acids 217-231) of beta-tubulin. J Biol Chem 270, 20235-20238.
Ringel, I., and Horwitz, S. B. (1991). Studies with RP 56976 (taxotere): a semisynthetic analogue of taxol. J Natl Cancer Inst 83, 288-291.
Rose, W. C., Clark, J. L., Lee, F. Y., and Casazza, A. M. (1997). Preclinical antitumor activity of water-soluble paclitaxel derivatives. Cancer Chemother Pharmacol 39, 486-492.
Rose, W. C., Fairchild, C., and Lee, F. Y. (2001a). Preclinical antitumor activity of two novel taxanes. Cancer Chemother Pharmacol 47, 97-105.
Rose, W. C., Lee, F. Y., Golik, J., and Kadow, J. (2000). Preclinical oral antitumor activity of BMS-185660, a paclitaxel derivative. Cancer Chemother Pharmacol 46, 246-250.
Rose, W. C., Long, B. H., Fairchild, C. R., Lee, F. Y., and Kadow, J. F. (2001b). Preclinical pharmacology of BMS-275183, an orally active taxane. Clin Cancer Res 7, 2016-2021.
Sampath, D., Discafani, C. M., Loganzo, F., Beyer, C., Liu, H., Tan, X., Musto, S., Annable, T., Gallagher, P., Rios, C., and Greenberger, L. M. (2003). MAC-321, a novel taxane with greater efficacy than paclitaxel and docetaxel in vitro and in vivo. Mol Cancer Ther 2, 873-884.
Sato, B., Muramatsu, H., Miyauchi, M., Hori, Y., Takase, S., Hino, M., Hashimoto, S., and Terano, H. (2000a). A new antimitotic substance, FR182877. I. Taxonomy, fermentation, isolation, physico-chemical properties and biological activities. J Antibiot (Tokyo) 53, 123-130.
Sato, B., Nakajima, H., Hori, Y., Hino, M., Hashimoto, S., and Terano, H. (2000b). A new antimitotic substance, FR182877. II. The mechanism of action. J Antibiot (Tokyo) 53, 204-206.
Schiff, P. B., Fant, J., and Horwitz, S. B. (1979). Promotion of microtubule assembly in vitro by taxol. Nature 277, 665-667.
Schiff, P. B., and Horwitz, S. B. (1980). Taxol stabilizes microtubules in mouse fibroblast cells. Proc Natl Acad Sci U S A 77, 1561-1565.
Shintani, Y., Tanaka, T., and Nozaki, Y. (1997). GS-164, a small synthetic compound, stimulates tubulin polymerization by a similar mechanism to that of Taxol. Cancer Chemother Pharmacol 40, 513-520.
Shionoya, M., Jimbo, T., Kitagawa, M., Soga, T., and Tohgo, A. (2003). DJ-927, a novel oral taxane, overcomes P-glycoprotein-mediated multidrug resistance in vitro and in vivo. Cancer Sci 94, 459-466.
Smart, C. R., Hogle, H. H., Robins, R. K., Broom, A. D., and Bartholomew, D. (1969). An interesting observation on nordihydroguaiaretic acid (NSC-4291; NDGA) and a patient with malignant melanoma--a preliminary report. Cancer Chemother Rep 53, 147-151.
Stachel, S. J., Chappell, M. D., Lee, C. B., Danishefsky, S. J., Chou, T. C., He, L., and Horwitz, S. B. (2000). On the total synthesis and preliminary biological evaluations of 15(R) and 15(S) aza-dEpoB: a Mitsunobu inversion at C15 in pre-epothilone fragments. Org Lett 2, 1637-1639.
Syed, S. K., Beeram, M., Takimoto, C. H., Jakubowitz, J., Kimura, M., Ducharme, M., Gadgeel, S., De Jager, R., Rowinsky, E., and Lorusso, P. (2004). Phase I and Pharmacokinetics (PK) of DJ-927, an oral taxane, in patients (Pts) with advanced cancers. J Clin Oncol (Meeting Abstracts) 22, 2028.
Takeda, Y., Yoshino, T., Uoto, K., Chiba, J., Ishiyama, T., lwahana, M., Jimbo, T., Tanaka, N., Terasawa, H., and Soga, T. (2003). New highly active taxoids from 9beta-dihydrobaccatin-9,10-acetals. Part 3. Bioorg Med Chem Lett 13, 185-190.
Taraboletti, G., Micheletti, G., Rieppi, M., Poli, M., Turatto, M., Rossi, C., Borsotti, P., Roccabianca, P., Scanziani, E., Nicoletti, M. I., et al. (2002). Antiangiogenic and antitumor activity of IDN 5390, a new taxane derivative. Clin Cancer Res 8, 1182-1188.
Tarrant, J. G., Cook, D., Fairchild, C., Kadow, J. F., Long, B. H., Rose, W. C., and Vyas, D. (2004). Synthesis and biological activity of macrocyclic taxane analogues. Bioorg Med Chem Lett 14, 2555-2558.
ter Haar, E., Kowalski, R. J., Hamel, E., Lin, C. M., Longley, R. E., Gunasekera, S. P., Rosenkranz, H. S., and Day, B. W. (1996). Discodermolide, a cytotoxic marine agent that stabilizes microtubules more potently than taxol. Biochemistry 35, 243-250.
Tinley, T. L., Randall-Hlubek, D. A., Leal, R. M., Jackson, E. M., Cessac, J. W., Quada, J. C., Jr., Hemscheidt, T. K., and Mooberry, S. L. (2003). Taccalonolides E and A: Plant-derived steroids with microtubule-stabilizing activity. Cancer Res 63, 3211-3220.
Todd, R., Sludden, J., Boddy, A. V., Griffin, M. J., Robson, L., Cassidy, J., Bisset, D., Main, M., Brannan, M. D., Elliott, S., et al. (2001). Phase I and Pharmalocological Study of CT-2103, a Poly(L-glutamic Acid)-Paclitaxel Conjugate. Proc Am Soc Clin Oncol 20, 111a (abstract 433).
Uyar, D., Takigawa, N., Mekhail, T., Grabowski, D., Markman, M., Lee, F., Canetta, R., Peck, R., Bukowski, R., and Ganapathi, R. (2003). Apoptotic pathways of epothilone BMS 310705. Gynecol Oncol 91, 173-178.
Wani, M. C., Taylor, H. L., Wall, M. E., Coggon, P., and McPhail, A. T. (1971). Plant antitumor agents. VI. The isolation and structure of taxol, a novel antileukemic and antitumor agent from Taxus brevifolia. J Am Chem Soc 93, 2325-2327.
Wolff, A. C., Donehower, R. C., Carducci, M. K., Carducci, M. A., Brahmer, J. R., Zabelina, Y., Bradley, M. O., Anthony, F. H., Swindell, C. S., Witman, P. A., et al. (2003). Phase I study of docosahexaenoic acid-paclitaxel: a taxane-fatty acid conjugate with a unique pharmacology and toxicity profile. Clin Cancer Res 9, 3589-3597.
Wu, J. H., Batist, G., and Zamir, L. O. (2001). Identification of a novel steroid derivative, NSC12983, as a paclitaxel-like tubulin assembly promoter by 3-D virtual screening. Anticancer Drug Des 16, 129-133.
Zhang, X., Burt, H. M., Mangold, G., Dexter, D., Von Hoff, D., Mayer, L., and Hunter, W. L. (1997a). Anti-tumor efficacy and biodistribution of intravenous polymeric micellar paclitaxel. Anticancer Drugs 8, 696-701.
Zhang, X., Burt, H. M., Von Hoff, D., Dexter, D., Mangold, G., Degen, D., Oktaba, A. M., and Hunter, W. L. (1997b). An investigation of the antitumour activity and biodistribution of polymeric micellar paclitaxel. Cancer Chemother Pharmacol 40, 81-86.
Saruhashi Y., Young W., Perkins R. (1996). Exp Neurol. 139, 203-213.
Antri M., Orsal D., Barthe JY. (2002). Eur J Neurosci. 16, 467-476
Kim J. E. , Liu B. P., Park J. H. , Strittmatter S. M. (2004). Neuron 44, 439-451.
Lee Y. S., Lin C. Y., Robertson R. T., Hsiao I., Lin V. W. (2004). J Neuropathol Exp Neurol. 63, 233-245.
Bareyre F. M., Kerschensteiner M., Raineteau O., Mettenleiter T. C., Weinmann O., and Schwab M. E. (2004). Nat Neurosci 7, 269-277.
Ishii K., Toda M., Nakai Y., Asou H., Watanabe M., Nakamura M., Yato Y., Fujimura Y., Kawakami Y., Toyama Y., Uyemura K. (2001). J Neurosci Res 65, 500-507.
Smith K. J. and Bennett B. J. (1987). J Anat 153, 203-215.
Tracey D. (1995). Ascending and descending pathways in the spinal cord. In The Rat Nervous System, G. Paxinos, ed. (San Diego, CA, Academic Press), pp. 67-80.
Paxinos G. and Watson C. (1982). The Rat Brain in Stereotaxic Coordinates, 1 nd ed. Academic Press.
Reiner A., Veenman C. L., Medina L., Jiao Y., Del Mar N., Honig M. G. (2000). J Neurosci Methods. 103, 23-37.
Schmidt B. J., and Jordan L. M. (2000). Brain Res Bull 53, 689-710.
Basso D. M., Beattie M. S., and Bresnahan J. C. (1995). J Neurotrauma 12, 1-21.
Bradbury E. J. et al. (2002). Nature 416, 636-640.
Moon L. D. et al. (2001). Nat. Neuroscience 4, 465-466.
Hermanns, S. et al. (2001). J. Neurosci. Methods 110, 141-146.
Nikulina, E. et al. (2004). Proc. Natl. Acad. Sci. USA 101, 8786-8790.
Pearse, D. D. et al. (2004). Nat. Med. 10, 610-616.
Goslin, K. and Banker, G. (1991). Rat hippocampal neurons in low-density culture. In Culturing Nerve Cells (Cambridge, MA; MIT Press, G. Banker and K. Goslin, eds.), pp. 251-281.
De Hoop, M. J. et al. (1998). Culturing hippocampal neurons and astrocytes from fetal rodent brain. In Cell Biology: A Laboratory Handbook, J. E. Celis, ed. (San Diego, CA: Academic Press), pp. 154-163.

## Claims

1. Use of one or more microtubule stabilizing compounds for the preparation of a pharmaceutical composition for the treatment of lesions of CNS axons wherein the pharmaceutical composition is administered locally directly into the lesion or immediately adjacent thereto, whereby the one or more compound(s) is/are selected from the group consisting of:
taxanes, epothilones, laulimalides, sesquiterpene lactones, sarcodictyins, diterpenoids, peloruside A, discodermolide, dicoumarol, ferulenol, NSC12983, taccalonolide A, taccalonolide E, Rhazinilam, nordihydroguaiaretic acid (NDGA), GS-164, borneol esters, Synstab A, Tubercidin and FR182877 (WS9885B).

2. The use according to claim 1, wherein the
(a) the taxane(s) is/are selected from the group consisting of IDN5190, IDN 5390, BMS-188797, BMS-184476, BMS-185660, RPR109881A, TXD258(RPR11625A), BMS-275183, PG-TXL, CT-2103, polymeric micellar paclitaxel, Taxoprexin, PTX-DLPC, PNU-TXL (PNU166945), MAC-321, Taxol, Protaxols, photoaffinity analogues of Taxol, photoactivatable Taxol and Docetaxel/Taxotere;
(b) the epothilone(s) is/are selected from the group consisting of epothilone A, epothilone B, dEpoB, BMS-247550 (aza-EpoB), epothilone D, dEpoF and BMS-310705;
(c) the sesquiterpene lactone(s) is/are Parthenolide or Costunolide;
(d) the sarcodictyin(s) is/are selected from sarcodictyin A and sarcodictyin B;
(e) the diterpenoid(s) is/are selected from the group consisting of Eleutherobins, caribaeoside and caribaeolin; or
(f) the discodermolide is/are a marine-derived polyhydroxylated alkatetraene lactone, used as a immunosuppressive compound.

3. The use according to claim 1 or 2, wherein the pharmaceutical composition is administered using an osmotic pump or by local syringe injection and/or said pharmaceutical composition comprises gelfoam, Elvax, liposomes, microspheres, nanoparticles and/or biodegradable polymers.

4. The use according to any one of claims 1 to 3, wherein the one or more taxane comprised in the pharmaceutical composition is/are in a final concentration in a range of 0,1 pM and 1mM.

5. The use according to any one of claims 1 to 4, wherein the pharmaceutical composition further comprises a compound selected from the group of c3-exoenzyme, chondroitinase, an iron chelator, cAMP or a derivative thereof such as dibutyryl cAMP, or a phosphodiesterase inhibitor such as rolipram.

6. A method for the treatment of lesions of CNS axons the method comprising the step of administering to a patient in the need thereof a pharmaceutical composition comprising
(i) one or more microtubule stabilizing compounds selected from the group consisting of taxanes, epothilones, laulimalides, sesquiterpene lactones, sarcodictyins, diterpenoids, peloruside A, discodermolide, dicoumarol, ferulenol, NSC12983, taccalonolide A, taccalonolide E, Rhazinilam, nordihydroguaiaretic acid (NDGA), GS-164, borneol esters, Synstab A, Tubercidin and FR182877 (WS9885B) and, optionally,
(ii) further comprising suitable formulations of carrier, stabilizers and/or excipients,
wherein the pharmaceutical composition is administered locally directly into the lesion or immediately adjacent thereto.

7. The method according to claim 6, wherein the
(a) the taxane(s) is/are selected from the group consisting of IDN5190, IDN 5390, BMS-188797, BMS-184476, BMS-185660, RPR109881A, TXD258(RPR11625A), BMS-275183, PG-TXL, CT-2103, polymeric micellar paclitaxel, Taxoprexin, PTX-DLPC, PNU-TXL (PNU166945), Protaxols, photoaffinity analogues of Taxol, photoactivatable Taxol and Docetaxel/Taxotere;
(b) the epothilone(s) is/are selected from the group consisting of epothilone A, epothilone B, dEpoB, BMS-247550 - (aza-EpoB), epothilone D, dEpoF and BMS-310705;
(c) the sesquiterpene lactone(s) is/are Parthenolide or Costunolide;
(d) the sarcodictyin(s) is/are selected from sarcodictyin A and sarcodictyin B;
(e) the diterpenoid(s) is/are selected from the group consisting of Eleutherobins, caribaeoside and caribaeolin; or
(f) the discodermolide is/are a marine-derived polyhydroxylated alkatetraene lactone, used as a immunosuppressive compound.

8. The method according to claim 6 or 7, wherein the pharmaceutical composition is administered using an osmotic pump or by local syringe injection and/or said pharmaceutical composition comprises gelfoam, Elvax, liposomes, microspheres, nanoparticles and/or biodegradable polymers.

9. The method according to any one of claims 6 to 8, wherein the one or more taxane comprised in the pharmaceutical composition is/are in a final concentration in a range of 0,1µM and 1mM.

10. The method according to any one of claims 6 to 9, further comprising the coadministration of a pharmaceutical composition comprising a compound selected from the group of c3-exoenzyme, chondroitinase, an iron chelator, cAMP or a derivative thereof such as dibutyryl cAMP, or a phosphodiesterase inhibitor such as rolipram and, optionally, further comprising suitable formulations of carrier, stabilizers and/or excipients.
